(19) Europäisches Patentamt
European Patent Office
Office européen des brevets

(11) **EP 4 685 464 A1**

(12) **EUROPÄISCHE PATENTANMELDUNG**

(43) Veröffentlichungstag:
    **28.01.2026  Patentblatt 2026/05**

(21) Anmeldenummer: **25191629.2**

(22) Anmeldetag: **24.07.2025**

(51) Internationale Patentklassifikation (IPC):
    **G01N 21/31** (2006.01)    **G01N 21/33** (2006.01)
    **G01N 21/3577** (2014.01)    **G01N 21/3581** (2014.01)
    **G01N 21/359** (2014.01)    **G01N 33/28** (2006.01)

(52) Gemeinsame Patentklassifikation (CPC):
    **G01N 21/31; G01N 33/2888;** G01N 21/33;
    G01N 21/3577; G01N 21/3581; G01N 21/359;
    G01N 33/2847

(84) Benannte Vertragsstaaten:
    **AL AT BE BG CH CY CZ DE DK EE ES FI FR GB GR HR HU IE IS IT LI LT LU LV MC ME MK MT NL NO PL PT RO RS SE SI SK SM TR**
    Benannte Erstreckungsstaaten:
    **BA**
    Benannte Validierungsstaaten:
    **GE KH LA MA MD TN**

(30) Priorität: **25.07.2024  DE 102024121265**

(71) Anmelder: **Rolls-Royce Solutions GmbH 88045 Friedrichshafen (DE)**

(72) Erfinder:
    • **Weidele, Horst 88709 Meersburg (DE)**

    • **Kottke, Thomas 71139 Ehningen (DE)**
    • **Fouquet, Marcel 88677 Markdorf (DE)**
    • **Rupp, David 88276 Berg (DE)**
    • **Osiecki, Annette 88048 Friedrichshafen (DE)**

(74) Vertreter: **Eisenführ Speiser Patentanwälte Rechtsanwälte PartGmbB Stralauer Platz 34 10243 Berlin (DE)**

(54) **VERFAHREN ZUR ZUSTANDSBESTIMMUNG EINES IN EINER MASCHINE BEFINDLICHEN BETRIEBSMITTELS, UND VORRICHTUNG AUSGEBILDET ZUR DURCHFÜHRUNG DES VERFAHRENS**

(57)    Die Erfindung betrifft ein Verfahren zur Zustandsbestimmung eines in einer Maschine (100) befindlichen Betriebsmittels (BM), insbesondere eines Betriebsöls (BÖ) und/oder Kühlmittels (KM), insbesondere mit einer Betriebsmittelwechseldetektion oder dergleichen Detektion eines Anfangsereignisses (EAn) oder eines Ereignisses (En), aufweisend die Schritte:
Zuführen des Betriebsmittels (BM) der Maschine zu einer an die Maschine (100) gekoppelten Spektroskopieeinrichtung (600),
Durchführen einer spektroskopischen Messanalyse mittels einer Messstrahlung der Spektroskopieeinrichtung (600),
Bestimmen eines spektroskopischen Messergebnisses (MY) basierend auf der spektroskopischen Messanalyse,
Setzen des spektroskopischen Messergebnisses (MY) in Relation zu einer Messreferenz (MY0), derart, dass aus der Relation ein auswertbares Messsignal (S) angegeben wird, wobei
- das spektroskopische Messergebnis (MY) über einen vorbestimmten, auf ein Anfangsereignis (EAn) in Bezug auf das Betriebsmittel (BM) folgenden Referenzbetriebszeitraum (R) wiederholt bestimmt wird unter Angabe von dem Referenzbetriebszeitraum (R) zugeordneten spektroskopischen Messergebnissen (MY), und aus den spektroskopischen Messergebnissen die Messreferenz (MY0) bestimmt wird, und
- das auswertbare Messsignal (S) über eine Betriebszeit (t) der Maschine (100) wiederholt angegeben wird, derart, dass ein bestimmtes Zustandsmerkmal (Z) für das Betriebsmittel (BM) signalisierbar ist.

EP 4 685 464 A1

1000

Fig. 1

**Beschreibung**

Die Erfindung betrifft ein Verfahren zur Zustandsbestimmung eines in einer Maschine befindlichen Betriebsmittels, insbesondere eines Betriebsöls oder eines Kühlmittels, insbesondere mit einer Betriebsmittelwechseldetektion oder Detektion eines Anfangsereignisses oder eines anderen Ereignisses. Die Erfindung betrifft auch eine Vorrichtung, vorzugsweise Steuer- und Messvorrichtung für eine Maschine oder eine Maschine mit der Steuer- und Messvorrichtung, ausgebildet zur Durchführung des Verfahrens.

In DE 10 2020 126 900 A1 ist ein Verfahren zum Erfassen eines Ölzustands eines Betriebsöls in einem Motor einer Brennkraftmaschine beschrieben. Dabei ist ein Ölleitsystem mit einem Ölfilter für das Betriebsöl und eine Öl-Differenzdruck-Messeinrichtung ausgebildet zum Erfassen einer Öldruck-Differenz oder dergleichen Öldruck-Relation des Betriebsöls über den Ölfilter im Ölleitsystem. Dazu wird auf dieser Basis eine Anzahl Relationsgrößen-Werte einer Trendentwicklung über Betriebsdauer-Zeitpunkte zugeordnet.

Zudem ist bekannt Betriebsmittel, insbesondere Ölproben im Labor mittels Infrarotspektrographen zu analysieren, um beispielsweise die Alterung des Betriebsmittels beurteilen zu können, wobei dazu eine Vergleichsprobe des frischen Betriebsmittels benötigt wird.

Ferner sind aus dem Stand der Technik Sensorsysteme bekannt, welche ebenfalls auf dem Verfahren der Infrarotspektrographie basieren, welche jedoch nicht den Wellenbereichsumfang wie Laborgeräte aufweisen. Um Ergebnisse zu ermitteln, arbeiten diese Sensorsysteme mit Vergleichsproben von Betriebsmitteln aus dem Labor und bestimmen anhand des Vergleichs vom Ist-Stand zum Soll-Stand die Werte der einzelnen Inhaltstoffe. Dies bezieht sich auf charakteristische Eigenschaften des Betriebsmittels, wie z. B. Säure- oder Basenzahl, insbesondere bei einem Betriebsöl. Die Vergleichsproben werden anhand eines Referenzmesssystems im Labor vermessen und die Referenzwerte werden typischerweise in einer Datenbank in den Sensorsystemen gespeichert.

Der Einsatz von Vergleichsproben ist jedoch bei im Betrieb befindlichen Maschinen, beispielsweise Verbrennungsmaschinen, insbesondere auf Schiffen, schwierig, da hier typischerweise oftmals Betriebsmittelsorten, insbesondere Ölsorten, verschiedener Hersteller gemischt werden. Ebenso sind diese Vergleichsproben oftmals nicht vorhanden oder die Zusammensetzung einer bestimmten Betriebsmittelsorte, insbesondere Ölsorte, hat sich im Laufe der Zeit geändert (z. B. durch Anpassung der Additive).

Außerdem ist die Zustandsbestimmung im Hinblick auf eine verlässliche Aussage für den weiteren Betrieb der Maschine, die unter Einsatz von Betriebsmitteln betrieben wird, durchaus herausfordernd und zudem systemkritisch. Entsprechende Messverfahren zur Detektion und Abgabe einer verlässlichen Aussage betreffend eine Notwendigkeit zum Betriebsmittelwechsel sind somit vergleichsweise aufwändig.

In DE 20 2007 019 631 U1 ist erläutert, dass der Verschleißgrad eines Öls, insbesondere eines Motoröls, mittels Infrarotspektroskopie bestimmt werden kann. Im mittleren Infrarotbereich finden sich Absorptionsbanden, die charakteristisch für den Säuregehalt, den Alkoholanteil oder den Wassergehalt sind. Insbesondere findet sich im Bereich von 2900-3000 nm ein Spektralbereich, der charakteristisch für den Wassergehalt eines Schmieröls ist. Mittels Infrarotdurchleuchtung eines Schmieröls kann daher festgestellt werden, wie hoch der Wasseranteil ist. Es wird eine Einrichtung zur Bestimmung des Wassergehalts in Mineralölen und ähnlichen Flüssigkeiten beschrieben, mit einer bestimmten Basenzahl und einem bestimmten Schwärzungsgrad mittels einer Infrarotmesszelle, die in einem Messkanal kontinuierlich von dem zu untersuchenden Mineralöl durchströmt wird und deren Ausgangsmesswert bei einem gewählten Spektralwert ein Maß für den Wassergehalt des Mineralöls bildet. Diese Schrift sieht zur Zustandsbestimmung eines in einer Maschine befindlichen Betriebsöls vor, dass eine Infrarotmesszelle als Durchströmküvette ausgebildet ist, bei der ein die Durchströmküvette durchleuchtender Infrarotstrahl von einem Infrarotempfänger erfasst wird, wobei der Ausgangsmesswert der Infrarotmesszelle mittels einer Recheneinheit zu einem gespeicherten Basiswert in Beziehung gesetzt wird, der im Wechsel zur Messung des zu untersuchenden Mineralöls mittels der Infrarotmesszelle und in bestimmten zeitlichen Abständen aus der Messung eines durch einen Spülkanal strömenden Referenzmineralöls erhalten wurde.

Der Ausgangsmesswert wird mittels der Recheneinheit und eines ersten Korrekturwerts kalibriert, welcher aus der jeweiligen Basenzahl des zu untersuchenden Mineralöls abgeleitet ist. Zur Erfassung des Schwärzungsgrads des zu untersuchenden Mineralöls ist ein für Weißlicht empfindlicher Fotoempfänger vorgesehen. Aus dem erfassten Schwärzungsgrad wird ein zweiter Korrekturwert abgeleitet, mit dessen Hilfe der erfasste Wassergehalt des zu untersuchenden Mineralöls rechnerisch verknüpft wird.

Ausgehend von den eingangs erläuterten bekannten Sensorsystemen, insbesondere der vorgenannten DE 20 2007 019 631 U1, setzen IR-basierte Betriebsmittel-Zustandsmonitorsysteme, insbesondere Betriebsöl-Zustandsmonitorsysteme, bislang immer eine Referenzgröße voraus, die entweder als Kalibrierwert Datenbank-basiert festgehalten wird oder wie in dem genannten Gebrauchsmuster separat mitzuführen ist.

Problematisch dabei ist der hohe Aufwand zur Pflege der Datenbank mit jedem verwendeten Betriebsmittel, insbesondere Betriebsöl, und dessen Parametern. Zudem kann bislang grundsätzlich ein unbekanntes bzw. unbestimmtes Betriebsmittel, insbesondere ein unbekanntes bzw. unbestimmtes Betriebsöl, nicht verwendet werden.

Besondere Probleme entstehen bei wechselnden Betriebsmittelsorten, insbesondere Betriebsölsorten, wie dies im Feld entweder durch Nachgeben von Betriebsmittel oder Betriebsmittelwechseln vorkommt. Dies ist auch problematisch bei zum Teil belastungs- oder umgebungsabhängigen Umständen. Dies trifft vor allem dann zu, wenn nur auf einen relativ statischen Referenzwert eines Zustands des Betriebsmittels erkannt wird. Dann kann ein Betriebsmittel-Zustand erst als kritisch angegeben werden, wenn eine Schädigung des Motors bereits droht.

An dieser Stelle setzt die Erfindung an, deren Aufgabe es ist, ein Verfahren und eine Vorrichtung anzugeben, insbesondere mit einer Überwachungseinrichtung, zur Zustandsbestimmung eines in einer Maschine befindlichen Betriebsmittels, mittels dem ein Betriebsmittel-Zustand flexibler und mit weniger Aufwand, gleichwohl bevorzugt verlässlicher oder jedenfalls rechtzeitig, detektierbar ist.

Das Verfahren und die Vorrichtung sollten Maßnahmen enthalten, mittels denen ein Betriebsmittel-Zustand einer Maschine oder eines Systems bzw. ein Verfahren zum Überwachen eines Betriebsmittel-Zustands in verbesserter Weise möglich wird. Insbesondere sollte eine entsprechend ausgebildete Steuerung, insbesondere eine Steuer- und Messvorrichtung, Steuermodule umfassen, mittels denen ein Warn- und/oder Alarm-Zustand für ein in einer Maschine befindliches Betriebsmittel flexibler und mit weniger Aufwand, gleichwohl verlässlicher oder jedenfalls rechtzeitig, detektierbar sind.

Diese Aufgabe wird in einem ersten Aspekt gelöst durch ein Verfahren gemäß Anspruch 1.

Das erfindungsgemäße Verfahren zur Zustandsbestimmung eines in einer Maschine befindlichen Betriebsmittels, weist die Schritte auf:

Zuführen des Betriebsmittels der Maschine zu einer an die Maschine gekoppelten Spektroskopieeinrichtung; Durchführen einer spektroskopischen Messanalyse mittels einer Messstrahlung der Spektroskopieeinrichtung; Bestimmen eines spektroskopischen Messergebnisses basierend auf der spektroskopischen Messanalyse; und Setzen des spektroskopischen Messergebnisses in Relation zu einer Messreferenz, derart, dass aus der Relation ein auswertbares Messsignal angegeben wird, wobei vorgesehen ist, dass

- das spektroskopische Messergebnis über einen vorbestimmten, auf ein Anfangsereignis in Bezug auf das Betriebsmittel folgenden Referenzbetriebszeitraum wiederholt bestimmt wird unter Angabe von dem Referenzbetriebszeitraum zugeordneten spektroskopischen Messergebnissen, und aus den spektroskopischen Messergebnissen die Messreferenz bestimmt wird, und

- das auswertbare Messsignal über eine Betriebszeit der Maschine wiederholt angegeben wird, derart, dass ein bestimmtes Zustandsmerkmal für das Betriebsmittel signalisierbar ist.

Die Aufgabe wird in einem zweiten Aspekt durch eine Vorrichtung gelöst, die ausgebildet ist zur Durchführung des Verfahrens.

Die Erfindung betrifft insbesondere eine Steuer- und Messvorrichtung für eine Maschine.

Die Erfindung betrifft insbesondere eine Maschine mit der Steuer- und Messvorrichtung.

Insbesondere wird mit dem genannten erfindungsgemäßen Verfahren und analog der Vorrichtung in vorteilhafter Weise eine Betriebsmittelwechseldetektion oder dergleichen Detektion eines Anfangsereignisses oder eines Ereignisses genutzt; das Anfangsereignis kann im Rahmen einer Weiterbildung zudem in vorteilhafter Weise bestimmt werden. Die Erfindung zeichnet sich zudem durch eine vorteilhafte Verfahrensweise bzw. eine technische Abfolge und einen Auswerteansatz aus, die es ermöglichen, die Messreferenz in vorteilhafter Weise zu bestimmen.

Die Erfindung hat erkannt, dass die Bestimmung der Messreferenz während des Betriebs der Maschine oder jedenfalls während das Betriebsmittel in der Maschine befindlich ist, erfolgen kann. Ein Anfangsereignis wie beispielsweise ein Betriebsmittelwechsel eignet sich, wie von der Erfindung erkannt, vorteilhaft dazu, im unmittelbaren zeitlichen Nachgang die Messreferenz zu bestimmen.

Eine aus dem Stand der Technik vermeintlich erforderliche --und zumeist unvorteilhaft "statische"-- Messreferenz wird nicht mehr benötigt. Das Konzept der Erfindung sieht vor, dass während des Betriebs oder jedenfalls während sich das Betriebsmittel in der Maschine befindet, ein Referenzwert zur Angabe der Messreferenz bestimmt wird. Dies führt dazu, dass eine Zustandsbestimmung eines in einer Maschine befindlichen Betriebsmittels möglich wird, mittels dem ein Betriebsmittel-Zustand flexibler und mit weniger Aufwand, gleichwohl bevorzugt verlässlicher oder jedenfalls rechtzeitig detektierbar ist.

Eine separat durchzuführende und separat von der Maschine zu erfolgende Kalibrierung wird ganz oder zumindest zu großen Teilen überflüssig. Stattdessen wird -ausgehend von dem Anfangsereignis-- ein Referenzbetriebszeitraum zur Bestimmung eines oder mehrerer Referenzwerte nach einem Betriebsmittelwechsel für die Bestimmung der Messreferenz genutzt. Dies führt zudem zu einer verlässlichen Messreferenz, ohne dass aufwändige Referenzmessungen oder Daten genutzt werden müssten.

Es zeigt sich, dass der --während des Betriebs oder jedenfalls während sich das Betriebsmittel in der Maschine befindet-- bestimmte Referenzwert zur Angabe der Messreferenz eben auch in der Lage ist, sich ändernde Betriebsmittelsorten oder Umstände vergleichsweise einfach zu berücksichtigen.

Da die Messreferenz verlässlicher ist, kann auch mit Vorteil eine verbesserte Auswertung des Messsignals über eine Betriebszeit der Maschine erfolgen. Eine Datenbank kann insofern entfallen; ein Betriebsmittelsortenwechsel bei Betriebsmittelwechsel wird grundsätzlich zulässig und ermöglicht gleichwohl eine nachfolgende verlässliche Zustandsbestimmung. Zudem entfällt eine chemische Vorabanalyse des Betriebsmittels, insbesondere in einem Labor.

Vorteilhafte Weiterbildungen der Erfindung sind den Unteransprüchen zu entnehmen und geben im Einzelnen vorteilhafte Möglichkeiten an, das oben erläuterte Konzept im Rahmen der Aufgabenstellung sowie hinsichtlich weiterer Vorteile zu realisieren.

Die Maschine ist insbesondere in Form einer Verbrennungsmaschine oder dergleichen Brennkraftmaschine, z. B. mit einem Motor in Form eines Dieselmotors oder Gas- oder sonstigen Otto-Motors, gebildet.

Als Betriebsmittel sind im Rahmen der vorliegenden Erfindung Fluide oder Stoffe im Allgemeinen, insbesondere Flüssigkeiten, zum Betreiben einer Maschine, insbesondere zur Schmierung und/oder Kühlung von Komponenten und/oder Teilbereichen der Maschine, zu verstehen. Im Rahmen der vorliegenden Weiterbildung sind als Betriebsmittel Betriebsöle und/oder Kühlmittel zu verstehen oder Betriebsmittel, die diese beinhalten. Vorteilhaft ist das Betriebsmittel in Form eines Betriebsöls und/oder eines Kühlmittels gebildet.

Unter Betriebsmittel sind vorzugsweise solche Fluide oder Stoffe im Allgemeinen zu verstehen, die in der Maschine zum Betrieb derselben geführt werden und die altern können bzw. deren Zustand sich verschlechtern kann, insbesondere also solche, die in der Maschine für eine relativ lange Betriebsdauer verbleiben, z. B. Betriebsmittel, die im Kreis geführt werden. Es können aber auch Betriebsmittel mit relativ langer Verweilzeit in der Maschine betroffen sein, die von extern zugeführt oder abgeführt werden und die in der Maschine für eine relative lange Betriebsdauer verbleiben.

Vorteilhaft ist vorgesehen, dass das spektroskopische Messergebnis für eine Referenzanzahl von Wiederholungen wiederholt bestimmt wird unter Angabe einer Referenzanzahl von dem Referenzbetriebszeitraum zugeordneten spektroskopischen Messergebnissen und der auf das Anfangsereignis in Bezug auf das Betriebsmittel folgende Referenz-Betriebszeitraum so festgelegt wird, dass die Referenzanzahl von spektroskopischen Messergebnissen im Referenz-Betriebszeitraum liegen. So kann im unmittelbaren zeitlichen Nachgang zum Anfangsereignis mit Vorteil festgelegt werden, in welchem Rahmen, d. h. Messumfang und/oder Zeitumfang, die Messreferenz zu bestimmen sein sollte.

Vorteilhaft wird geprüft, dass für die Referenzanzahl von Wiederholungen das spektroskopische Messergebnis im Wesentlichen unverändert ist, insbesondere die Referenzanzahl spektroskopischer Messergebnisse in einem vorbestimmten Konstantbereich liegt, wobei aus der Referenzanzahl der im Wesentlichen unveränderten spektroskopischer Messergebnisse des Referenz-Betriebszeitraums die Messreferenz bestimmt wird. Beispielsweise kann dies etwa durch Messen eines konstanten Niveaus einer Intensität für einen bestimmten Referenz-Betriebszeitraum umgesetzt werden. Dafür sind zumeist einige wenige Wiederholungen einer Bestimmung von spektroskopischen Messergebnissen ausreichend. Beispielsweise kann ein sich einpegelnder Intensitätswert bzw. eines Messspektrums einer Messung als Referenzintensität bzw. Referenzspektrum zur Bestimmung der Messreferenz genutzt werden.

Vorteilhaft ist vorgesehen, dass das spektroskopische Messergebnis mit Messstrahlung eines vorbestimmten Spektrums für wenigstens eine Messstrahlungslinie, eine Anzahl von Messstrahlungslinien oder für ein Spektrumsband der Messstrahlung zur Verfügung gestellt wird. So kann beispielsweise das spektroskopische Messergebnis eine mit der Messstrahlung aus der spektroskopischen Messanalyse am Betriebsmittel gewonnene Intensität sein. Die Intensität kann spektral aufgelöst sein. Es kann also ein Spektrum als ein spektroskopisches Messergebnis zur Verfügung stehen oder jedenfalls eine Anzahl geeigneter oder relevanter spektroskopischen Messstrahlungslinien bei bestimmten Frequenzen (oder Wellenlängen) aus diesem.

Typische mögliche Messaufzeichnungen mit in-situ Infrarotspektrographie betreffen, insbesondere bei einem Betriebsöl als Betriebsmittel:

- Anteil an Wasser
- Oxidation, Nitration und/oder Sulfation von Anteilen des Betriebsmittels
- Anteil an Additiven wie Phenol
- Anteil an Additiven wie ZDDP (Zinkdialkyldithiophosphat)
- Anteil an Carbonaten (insbesondere zur Bestimmung der TBN)
- Anteil an Aromatischer Phosphorestersäure
- Anteil an Aminischen Antioxidantien
- Grad an CH-Biegeschwingungen

ZDDP (Zinkdialkyldithiophosphat) ist ein gegen Verschleiß vor allem von Betriebsöl wirkendes Mittel.

Die TBN oder Gesamtbasenzahl gibt den Alkalitätsgrad eines Schmierstoffs an, wie etwa bei Betriebsöl. TBN ist ein Faktor für die Kontrolle und Verwaltung einer Öllebensdauer. Die Verwendung der TBN hilft bei der Neutralisierung von Säuren, die sich im Betrieb bilden. Z. B. sollten Kurbelgehäuseöle während des Betriebs einen angemessenen TBN-Wert beibehalten, um die Ansammlung von Säuren zu verhindern. Metallhaltige Detergenzzusätze sind eine Hauptquelle für die TBN in einem Schmierstoff. Ein wichtiger Test ist die Messung der TBN in Motorenölen. Dabei wird der zentrale anorganische Kern aus basischem Kalziumkarbonat/- hydroxid berücksichtigt, der im Schmierstoff durch Detergenzien-Seifenmoleküle in kolloidaler Suspension gehalten wird.

Analog lassen sich Messaufzeichnungen mit in-situ Infrarotspektrographie, insbesondere bei einem Kühlmittel oder anderen Betriebsmitteln, angeben.

Das auswertbare Messsignal wird bevorzugt über eine Betriebszeit des Betriebs der Maschine transient oder laufend wiederholt angegeben.

Das spektroskopische Messergebnis kann einem optischen Filter unterworfen werden und/oder das auswertbare Messsignal kann einem numerischen Filter unterworfen werden. Solche Maßnahmen erhöhen die Selektivität und verbessern die Auswertbarkeit des spektroskopischen Messergebnisses.

Im Rahmen einer besonders bevorzugten Weiterbildung wird das auswertbare Messsignal über die Betriebszeit der Maschine zur Darstellung als Verlauf angegeben, wobei mittels des Verlaufs, ein bestimmter Zustand des Betriebsmittels signalisierbar ist. Dies hat den Vorteil, dass eine Analyse und ein Abgleich zu Alarmschwellen aber vor allem Fehlergrenzen in verbesserter Weise möglich sind. Auch lassen sich Ereignisse anhand eines Verlaufs, der ggfs. zwischen einzelnen Messergebnissen interpoliert wird, in verbesserter Weise ermitteln.

Vorteilhaft ist das Anfangsereignis in Bezug auf das Betriebsmittel ein zum Bestimmen der Messreferenz bestimmtes oder geeignetes Anfangsereignis; insbesondere ist in Bezug auf das Betriebsmittel das Anfangsereignis ausgewählt aus der Gruppe von: ein Betriebsmittelwechsel, eine Betriebsmittelnachfüllung. Ein Betriebsmittelwechsel kann auch festgestellt werden, indem er von extern angezeigt wird. Mit Vorteil ist im Rahmen einer Weiterbildung vorgesehen, dass das Anfangsereignis in Bezug auf das Betriebsmittel festgestellt wird, indem geprüft wird, dass das Messsignal für ein Ereigniszeitfenster eine sprunghafte Änderung aufweist.

Insbesondere kann eine der sprunghaften Änderung zugeordnete sprunghafte Änderungsamplitude einen ersten Ereignis-Schwellwert überschreiten, insbesondere das Anfangsereignis oder generell ein Ereignis als ein Betriebsmittelwechsel identifiziert werden. Insbesondere kann eine der sprunghaften Änderung zugeordnete sprunghafte Änderungsamplitude einen zweiten Ereignis-Schwellwert überschreiten, insbesondere das Anfangsereignis oder generell ein Ereignis als eine Betriebsmittelnachfüllung identifiziert werden.

Mit Vorteil ist im Rahmen einer Weiterbildung vorgesehen, dass der erste Ereignis-Schwellwert den zweiten Ereignis-Schwellwert überschreitet. Anders ausgedrückt, ist damit die Erkenntnis verbunden, dass ein großer Sprung in den Messdaten zu Werten, die sich in Richtung Neubetriebsmittelzustand verändern, einen "Betriebsmittelwechsel" bedeuten, ein kleiner Sprung zu besseren Messwerten --in Richtung Neubetriebsmittelzustand hin-- bedeutet, dass "Betriebsmittel nachgefüllt wurde".

Im Falle der Feststellung des Anfangsereignisses oder generell eines Ereignisses ist vorteilhaft vorgesehen, dass

- wenigstens eine Betriebszeitangabe zum Ereigniszeitfenster festgehalten wird, und/oder
- wenigstens eine spektral selektive Information des spektroskopischen Messergebnisses festgehalten wird für das auswertbare Messsignal für das Ereigniszeitfenster.

Die wenigstens eine spektral selektive Information wird bevorzugt im Hinblick auf das vorbestimmte Spektrum der Messstrahlung ausgewertet.

Die Messstrahlung kann insbesondere eine Messstrahlungslinie oder ein Messspektrumsband umfassen, betreffend eine oder mehrere der Parameter ausgewählt aus der Gruppe bestehend aus: spektrale Position, spektrale Breite, Amplitude, Gradient und Phase der Messstrahlung, und Relationen der Parameter zueinander, insbesondere Relationen unterschiedlicher spektraler Positionen.

Bevorzugt ist die wenigstens eine spektral selektive Information selektiv für Additive, insbesondere Zink, Barium, Bor, Calcium, Magnesium oder Phosphor, und/oder Sulfate oder chemische Verbindungen in dem Betriebsmittel, insbesondere in dem Betriebsöl.

Vorteilhaft liegt die Messstrahlung im Infrarot (IR)-Bereich des optischen Spektrums, d. h. insbesondere ausgerichtet auf IR-aktive Substanzen, insbesondere im Bereich zwischen 700nm bis 12 $\mu$m. Die spektroskopische Messanalyse ist nicht beschränkt auf --gleichwohl bevorzugt auf-- eine IR-Spektroskopie ausgerichtet - möglich ist insofern zusätzlich oder alternativ auch eine spektroskopische Messanalyse mittels X (Röntgen), UV, VIS oder NIR- oder FIR-Spektroskopie; möglich ist auch eine THz-Spektroskopie. Der Begriff Spektroskopie ist insofern weit zu fassen als eine Untersuchung mit elektro-magnetischer Strahlung und sollte die auszuwertenden Ereignisse feststellbar machen.

Es könnte insofern nicht nur IR-Spektroskopie, sondern --zusätzlich oder alternativ-- auch eine, UV, VIS oder NIR- oder FIR Spektroskopie, zur Anwendung kommen. Auf diese Weise könnten nicht nur IR-aktive Substanzen im Betriebsmittel festgestellt werden, sondern auch --zusätzlich oder alternativ-- nicht-IR-aktive Substanzen, wie Elemente oder nicht-IR-aktive Moleküle.

Metalle beispielsweise könnten weniger mit IR-Spektroskopie vermessbar sein. Für die Messung von Molekül- oder Gitter-Schwingungen eignet sich insbesondere die FIR- oder THz-Spektroskopie. Außerdem könnten spezifische spektroskopische Sensoren für Metallpartikel genutzt werden; für die Messung von Gitter-Schwingungen eignet sich insbesondere die THz-Spektroskopie. Generell eignet sich THz-Spektroskopie mit Vorteil etwa für eine Messung an einem Volumenstrom; bevorzugt in Reflexion. Auch könnten zusätzlich oder alternativ spektroskopische Sensoren für Partikel kleiner als ca. 300 Mikrometer genutzt werden.

Im Rahmen einer besonders bevorzugten Weiterbildung ist vorgesehen, dass zum wiederholt angegebenen auswertbaren Messsignal über die Betriebszeit eine obere und/oder untere Fehlergrenze angegeben wird. Insbesondere kann mit Vorteil über einen Verlauf des auswertbaren Messsignals über die Betriebszeit die obere und/oder untere Fehlergrenze als Fehlergrenzenverlauf angegeben werden, die einen zu bevorzugenden Verlauf des auswertbaren Messsignals vorgeben.

Mit dem Begriff "einen zu bevorzugenden Verlauf des auswertbaren Messsignals vorgeben" ist im Prinzip eine generelle "Tendenz" oder ein "Trend" gemeint. Dies kann bevorzugt anhand der erwarteten Alterung des Betriebsöls vorberechnet werden. Mathematisch kann dies mittels einer prognostizierten oder dergleichen anzunehmenden Amplitude und mit einer Steigung oder einem ggfs. gleichbleibendem Abstandsmaß umgesetzt werden, das für ein wiederholt angegebenes auswertbares Messsignal über eine Betriebszeit, insbesondere dessen Verlauf, gelten sollte - das Abstandsmaß kann als Verlauf gemittelter Werte oder mit einer extrapolierten Grenzkurve angebbar sein. Insofern meint "einen zu bevorzugenden Verlauf des auswertbaren Messsignals vorgeben" eher ein mit bestimmter Fehlerabweichung bestenfalls anzunehmendes Trendband.

Im Rahmen dieser Weiterbildung wurde insbesondere erkannt, dass ein das Mitführen eines Trends, z. B. eines Trendbandes umfassend eine obere und/oder untere Fehlergrenze, eine normale Betriebsmittel-Alterungen oder Einflüsse berücksichtigen kann - in seinem Abstandsmaß zum tatsächlichen Verlauf; anders ausgedrückt, die normalerweise zu erwartenden Abweichungen und/oder Fehler berücksichtigt. Dies kann etwa eine Abweichung durch Wassereintrag im Rahmen des normalen Gebrauchs sein oder im Rahmen des normalen Gebrauchs auch ein Dieseleintrag.

Die Spektroskopieeinrichtung ist vorteilhaft Teil einer komplexeren Messeinrichtung. Insbesondere kann die Messeinrichtung eine weitere Messeinheit umfassen, wie eine Messeinheit ausgebildet zur Bestimmung einer Temperatur, Viskosität, Trübung oder Dichte des Betriebsmittels.

Insbesondere kann die obere und/oder untere Fehlergrenze zum Messsignal auf Grundlage einer Anzahl von Messparametern des Betriebsmittels und/oder der Maschine und/oder eine Betriebsumgebung der Maschine bestimmt werden. Insbesondere kann zum spektroskopischen Messergebnis wenigstens ein weiterer Betriebsmittelzustandsparameter unabhängig bestimmt werden, insbesondere für den Referenz-Betriebszeitraum, der ausgewählt ist aus der Gruppe bestehend aus: Temperatur, Viskosität, Trübung, Dichte.

Im Rahmen einer besonders bevorzugten Weiterbildung ist vorgesehen, dass das bestimmte Zustandsmerkmal des Betriebsmittels signalisierbar ist im Hinblick auf ein Ereignis, ausgewählt aus der Gruppe bestehend aus: einem Betriebsmittelwechsel, einer Betriebsmittelnachfüllung, einem Wassereintrag in das Betriebsmittel, einem Kraftstoffeintrag in das Betriebsmittel, einem Eintrag von Verschmutzung und/oder Ruß in das Betriebsmittel.

Ausführungsformen der Erfindung werden nun nachfolgend anhand der Zeichnung im Vergleich zum Stand der Technik, welcher zum Teil ebenfalls dargestellt ist, beschrieben. Diese soll die Ausführungsformen nicht notwendigerweise maßstäblich darstellen, vielmehr ist die Zeichnung, wo zur Erläuterung dienlich, in schematisierter und/oder leicht verzerrter Form ausgeführt. Im Hinblick auf Ergänzungen der aus der Zeichnung unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in der Zeichnung sowie in den Ansprüchen offenbarte Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, der Zeichnung und/oder den Ansprüchen offenbarten Merkmale.

Die allgemeine Idee der Erfindung ist nicht beschränkt auf die exakte Form oder das Detail der im Folgenden gezeigten und beschriebenen, bevorzugten Ausführungsform oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Weitere Vorteile, Merkmale und Einzelheiten der Erfindung ergeben sich aus der nachfolgenden Beschreibung der bevorzugten Ausführungsformen sowie anhand der Zeichnung; diese zeigt in:

FIG. 1          ein Schema einer Maschine, die unter Einsatz eines Mineralöls betrieben wird, mit einem Steuer- und Messsystem in einer bevorzugten Ausführungsform;

FIG. 2          in Ansicht (A) ein exemplarisch dargestellter Signalverlauf über einen Referenz-Betriebszeitraum einer Betriebsdauer einer Maschine, die unter Einsatz eines Mineralöls betrieben wird, nach einem Anfangsereignis --in diesem Fall ein Ölwechsel bei einer Brennkraftmaschine, d. h. mit einem Verbrennungsmotor--, wobei das Anfangsereignis wie vorliegend ein Ölwechsel im Signalverlauf als Ereignis erkennbar ist und genutzt wird zur Bestimmung der Messreferenz; in Ansicht (B) ein Ablaufdiagramm für einen Verfahrensablauf zur Bestimmung der Messreferenz;

FIG. 3 ein exemplarisch dargestellter Signalverlauf über die Betriebszeit einer Betriebsdauer einer Maschine --in diesem Fall eine Brennkraftmaschine, d. h. mit einem Verbrennungsmotor--, die unter Einsatz eines Mineralöls betrieben wird, wobei bestimmte Ereignisse wie vorliegend ein Ölwechsel und eine Ölnachfüllung im Signalverlauf als Ereignis erkennbar sind;

FIG. 4 ein Ablaufdiagramm für eine erste Ausführungsform eines Ablaufs eines Verfahrens zur Ölwechseldetektion in einer ersten Variante;

FIG. 5 einen exemplarischen Signalverlauf analog zu FIG. 3, wobei ein Wassereinbruch im Signalverlauf als Ereignis erkennbar ist und der Signalverlauf eine untere Fehlergrenze eines Trendbandes unterschreitet zur Angabe eines Warnhinweises;

FIG. 6A ein Ablaufdiagramm für eine erste Ausführungsform eines Ablaufs eines Verfahrens zur Angabe eines Alarmhinweises;

FIG. 6B ein Ablaufdiagramm für eine erste Ausführungsform eines Ablaufs eines Verfahrens zur Angabe eines Warnhinweises;

FIG. 7A, FIG. 7B ein exemplarisch gezeigter Signalverlauf analog zu FIG. 5 mit einem gem. FIG. 7A erkennbaren mitgeführten Trendband von Fehlergrenzen und einem gem. FIG. 7B erkennbaren Ereignis im Signalverlauf, das auf einen Motorschaden schließen lässt und wobei der Signalverlauf eine untere Fehlergrenze eines Trendbandes unterschreitet zur Angabe eines Warnhinweises;

FIG. 8A, FIG. 8B ein exemplarisch gezeigter Signalverlauf analog zu FIG. 1 mit einem mitgeführten Trendband von Fehlergrenzen und wobei zudem ein Fremdstoffeintrag (FIG. 8A) und ein Lufteintrag (FIG. 8B) im Signalverlauf als Ereignis erkennbar ist und der Signalverlauf eine untere Fehlergrenze eines Trendbandes unterschreitet (FIG. 8A), bzw. der Signalverläufe eine obere Fehlergrenze eines Trendbandes überschreitet (FIG. 8B) zur Angabe eines Warnhinweises;

FIG. 9 schematisch ein Steuer- und Messsystem im Detail mit der aufgezeigten Funktionalität zur Bestimmung eines Ölzustands gemäß einer bevorzugten Weiterbildung.

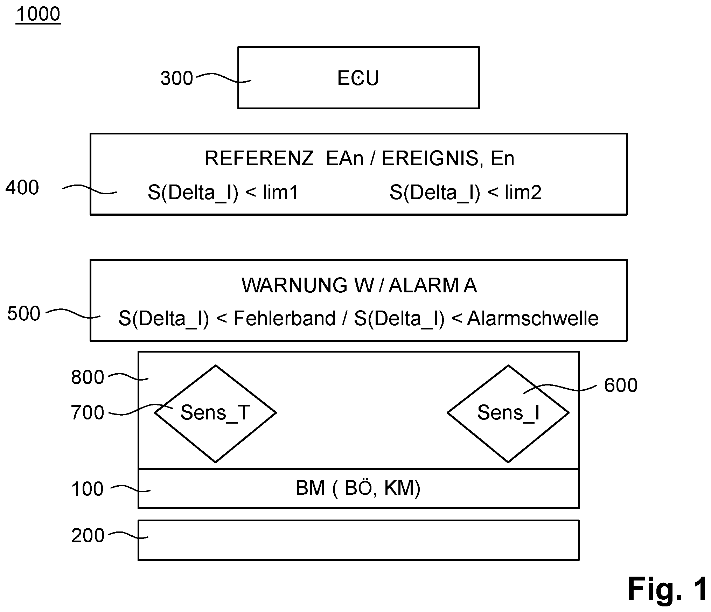

FIG. 1 zeigt schematisch in einer Übersicht eine bevorzugte Ausführungsform einer Vorrichtung 1000 gemäß dem Konzept der Erfindung als System aus einer Steuereinheit 300 und Messvorrichtung an einer Maschine 100 und mit einer entsprechenden Einrichtung 200 zur Signalisierung bzw. zum Überwachen oder dgl. zur Angabe bzw. Anzeige eines Zustandes eines in der Maschine befindlichen Betriebsmittels BM, wie etwa eines Kühlmittels KM oder eines Betriebsöls BÖ.

Im Folgenden wird das Konzept der Erfindung anhand eines nicht einschränkenden Ausführungsbeispiels für ein Betriebsöl BÖ in der Maschine 100 erläutert. Dabei ist zu verstehen, dass sich die damit beschriebenen und besonders bevorzugten Merkmale im Hinblick auf ein Betriebsöl BÖ als besonders relevant und vorteilhaft erwiesen haben im Rahmen des Konzepts der Erfindung. Gleichwohl ist zu verstehen, dass die beschriebenen Merkmale wie erläutert oder analog auch im Hinblick auf ein anderes Betriebsmittels BM, wie etwa ein Kühlmittel KM, mit den genannten Vorteilen relevant sein können. Dieses vorrausgeschickt, wird das Konzept der Erfindung anhand des nicht einschränkenden Ausführungsbeispiels für ein Betriebsöl BÖ in der Maschine 100 erläutert und ist m. E. insofern übertragbar auch auf andere Betriebsmittel BM, wie etwa ein Kühlmittel KM, in der Maschine 100.

Im Einzelnen ist dazu in FIG. 1 zunächst schematisch das System der Vorrichtung 1000 mit einer Maschine 100 gezeigt, die vorliegend im Rahmen einer bevorzugten Ausführungsform nicht einschränkend beschrieben ist anhand einer Brennkraftmaschine. Diese wird vor allem zur Schmierung des Motors derselben und dessen Komponenten wie Kolben, Pleuel und Kurbel- und Nockenwellen mit einem Betriebsmittel BM, vorliegend Betriebsöl BÖ, betrieben. Grundsätzlich kann im Rahmen des Konzepts der Erfindung auch eine andere Maschine umfasst sein, die mit dem in der Maschine befindlichen Betriebsmittel BM zu betreiben ist. Beispielsweise gilt die nachfolgende Beschreibung der Zeichnung gleichermaßen für eine Arbeitsmaschine oder Maschinen im Bereich der Energieerzeugung sowie zusätzlich oder alternativ auch für deren Getriebe oder Getriebe als solche - auch diese Art Maschinen sind mit in der Maschine jeweils befindlichen Betriebsmittel BM zu betreiben, insbesondere zur Schmierung der drehenden Teile und auch gegebenenfalls Lagerung derselben.

Die Maschine 100 ist vorliegend mit einer Sensorik ausgestattet, die hier maßgeblich eine Sensorik oder dgl. Analyseeinrichtungen einer Spektroskopieeinrichtung 600 umfasst, die gemäß der vorliegenden Ausführungsform wenigstens einen Intensitätssensor Sens_I umfasst.

Die Spektroskopieeinrichtung 600 ist in diesem Ausführungsbeispiel vorteilhaft Teil einer komplexeren Messeinrichtung 800. Insbesondere kann die Messeinrichtung 800 eine weitere Messeinheit umfassen, wie eine Messeinheit 700 ausgebildet zur Bestimmung einer Temperatur, die hier einen Temperatursensor Sens_T aufweist. Auch können weitere Messeinheiten ausgebildet zur Bestimmung einer Viskosität, Trübung oder Dichte des Betriebsmittels BM vorgesehen sein.

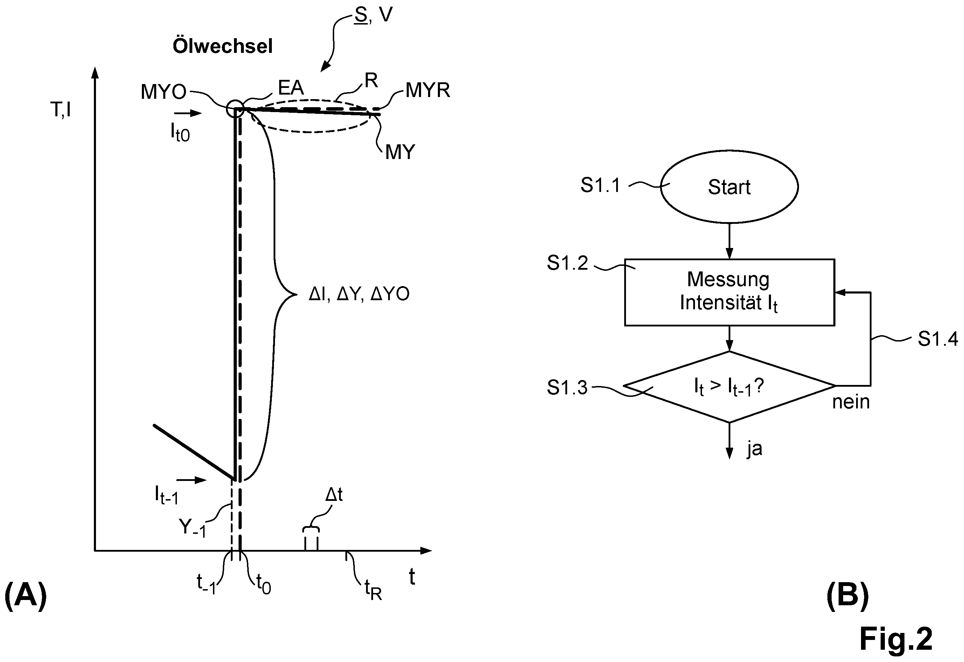

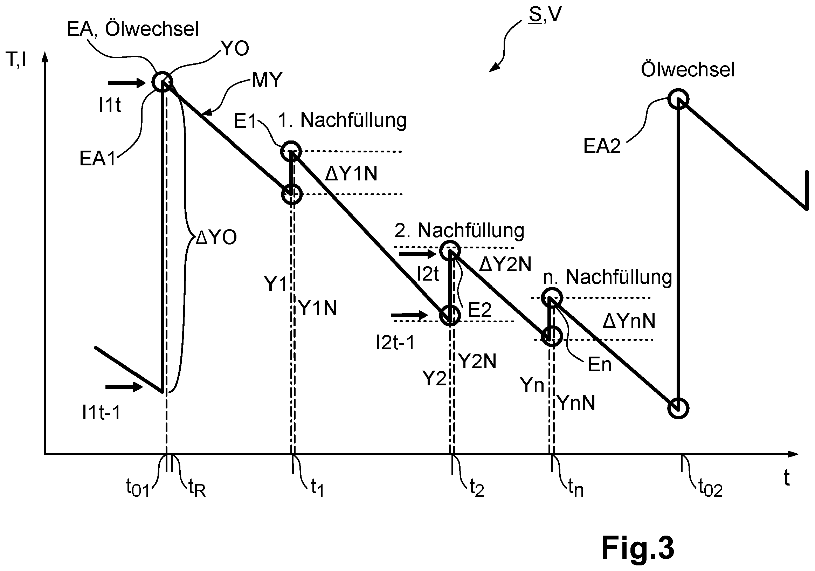

Die Spektroskopieeinrichtung 600 mit wenigstens dem Intensitätssensor Sens_I sowie die ggfs. weiteren Komponenten einer Analyseeinrichtung sind ausgebildet, das auswertbare Messsignal S bevorzugt über eine Betriebszeit des Betriebs der Maschine 100 transient oder laufend wiederholt anzugeben. Insbesondere wird in-situ über eine sehr lange Zeit des Betriebszeitraums, in der Regel über die gesamte Motorlaufzeit, in regelmäßigen, zeitlich kürzeren Intervallabständen, gemessen. Die Intervallabstände werden unter anderem unter Berücksichtigung der gewünschten oder technisch möglichen zeitlichen Auflösung, etwa entsprechend einem möglichst kleinem oder angemessenem Zeitschritt $\Delta t$ gewählt. Zudem kann ein zu überwachender Betriebszeitraum --z. B. die Betriebszeit t (u. a. FIG. 2 Ansicht (A), FIG. 3), nach der spätestens ein Fehler oder Ölwechsel im Sinne eines messbaren Ereignisses E erkannt werden sollte-- angemessen festgelegt werden.

Die Spektroskopieeinrichtung 600 mit wenigstens dem Intensitätssensor Sens_I kann in der Maschine 100 selbst oder in dessen Peripherie angeordnet sein und jedenfalls über einen maßgeblichen Betriebszeitraum zum Durchführen einer spektroskopischen Messanalyse des Betriebsöls BÖ mittels einer Messstrahlung am Betriebsöl BÖ mit der Maschine 100 gekoppelt sein.

Die Maschine 100, vorliegend in Form einer Brennkraftmaschine, ist auch mit weiterer Sensorik ausgestattet, wie vorliegend wenigstens einer Temperatursensorik Sens_T, zur Bestimmung einer Öltemperatur des Betriebsöls BÖ. Die Maschine 100 ist vorliegend gemäß dem Konzept der Erfindung ausgebildet mit der Spektroskopieeinrichtung Sens_I zum Bestimmen eines spektroskopischen Messergebnisses basierend auf der spektroskopischen Messanalyse des Betriebsöls BÖ.

Da ein solches spektroskopisches Messergebnis regelmäßig von der Temperatur des Betriebsöls BÖ abhängig ist, wird dieses also unter Berücksichtigung der entsprechenden Betriebsöltemperatur angegeben; zur Erlangung vergleichbarer Messergebnisse werden die spektroskopischen Messergebnisse bei möglichst gleichbleibender Temperatur bestimmt. Insgesamt wird das Messergebnis unter Berücksichtigung der durch die Spektroskopieeinrichtung Sens_I beispielsweise bestimmten Intensität I der spektroskopischen Messanalyse und der durch die Temperatursensorik Sens_T bestimmten Temperatur angegeben. Tatsächlich sollte zur Messfehlervermeidung eine Korrelation des spektroskopischen Messergebnisses mit der Temperatur des Betriebsöls BÖ berücksichtigt werden. Die Signalintensitäten, vor allem einer Infrarotmessung, sind regelmäßig abhängig von einer Öltemperatur.

Die Signalintensität bei einer Transmissionsmessung T steigt bei zunehmender Öltemperatur an (bei Reflexionsmessung fällt sie). Beispielsweise sind bei einer Frischölzufüllung --wie sie in Bezug auf FIG. 3 erläutert ist-- Sprünge der Signalintensität I bei einer Transmissionsmessung T kleiner als beim Ölwechsel. Daher sollte das Betriebsöl BÖ erst wieder auf Betriebstemperatur erwärmt werden, um dies messtechnisch sicher zu erfassen; dies kann über eine Temperaturmessung kontrolliert werden.

Es können dennoch ausgefilterte Messwerte nicht verworfen werden, sondern separat gespeichert werden; z. B. in entsprechenden Arrays abgelegt werden (bzw. können auch alle Werte in einer Dateieinheit oder dgl. File/Array oder anders in geeigneter Weise abgelegt sein. Diese könnten zur Auswertung anhand Temperaturfenster gefiltert werden; z. B. entsprechend einem Temperaturschema in 5° Schritten.

Solche den Temperaturen zugeordnete Werte werden dann jeweils als eigene Reihen ausgewertet. Diese Reihen sind gegeneinander in der "y-Achse" verschoben, aber sollten dieselben Tendenzen aufweisen.

Grundsätzlich kann ein spektroskopisches Messergebnis auch anderen physikalisch und/oder messtechnisch begründeten Filtern unterworfen werden, wie dem hier genannten Temperaturfilter. Auch optische Filter, die hier nicht im Einzelnen erläutert sind, haben sich aus Gründen der Selektivität als vorteilhaft erwiesen.

Wenn nach einer geeigneten -wie zuvor beispielhaft erläutert- physikalischen und/oder messtechnischen Filteranwendung die vorgenannten Tendenzen nicht gleichartig sein sollten, kann darauf geschlossen werden, dass das Betriebsöl BÖ verschlissen ist. Dies ist plausibel, da in solchen Fällen ein Rußanteil im Betriebsöl BÖ signifikant wird - dies kann auch anhand einer breitbandigen spektroskopischen Messung überprüft werden. Betriebsöl BÖ ist bei höheren Temperaturen lichtdurchlässiger, dieser Effekt ist bei einer Verrußung nicht mehr vollständig gegeben.

Auch die erläuterten numerischen Filtermethoden sind nur beispielhaft genannt; andere Vorgehensweisen numerischer Umsetzung sind möglich. Möglich ist beispielsweise eine oben genannte oder dergleichen Verschiebung oder Streckung, insbesondere Normierung, des Signals. Möglich ist auch eine Glättung, insbesondere Mittelung, oder Inter- oder Extrapolation der spektroskopischen Messergebnisse zur Angabe des Signals. Die spektroskopischen Messergebnisse können auch einem numerischen Frequenzfilter unterworfen werden.

Insgesamt kann ein so oder anders in geeigneter Weise aufbereitetes und damit auswertbares Messergebnis der spektroskopischen Messanalyse über einen Kommunikations-BUS oder dergleichen Signalisierungseinrichtung zur Auswertung weitergegeben werden oder auf einer geeigneten Monitoreinrichtung oder einer Schnittstelle zur weiteren Analyse zur Verfügung gestellt werden.

Insbesondere ist eine hier schematisch dargestellte Einrichtung 200 zur Signalisierung bzw. zum Überwachen des basierend auf der spektroskopischen Messanalyse bestimmten spektroskopischen Messergebnisses ausgebildet, dieses an eine Steuereinheit 300 zu kommunizieren. Die Einrichtung 200 kann über entsprechende Datenschnittstellen und Datenwege verfügen, die der Maschine 100 zugeordnet sind bzw. an diese datenübertragend angebunden sind.

Die Steuereinheit 300 ist ausgebildet, die Temperatursensorik Sens_T und die Spektroskopieeinrichtung 600 mit wenigstens dem Intensitätssensor Sens_I derart zu steuern, dass diese ein auswertbares Messsignal S angeben. Das auswertbare Messsignal S sollte über eine Betriebszeit der Maschine 100 wiederholt angegeben werden, so dass darüber mittels der Einrichtung 200 ein bestimmtes Zustandsmerkmal Z für das Betriebsöl BÖ signalisierbar ist.

Es zeigt sich, wie eingangs erläutert, dass --ein in FIG. 2 Ansicht (A) oder FIG. 3 beispielhaft und näher erläutertes-- spektroskopisches Messergebnis MY, beispielsweise eine geeignete Darstellung einer Intensität I, in Relation zu setzen ist, zu einer Messreferenz MY0, beispielsweise einer Präferenzintensität I0, derart, dass aus eben jener Relation ein auswertbares Messsignal S anzugeben ist.

Gemäß der in FIG. 1 gezeigten Ausführungsform ist ein erstes Steuermodul 400 vorgesehen, das ausgebildet ist, das spektroskopische Messergebnis MY auszuwerten im Hinblick auf die Bestimmung einer Messreferenz MY0 und/oder ein Anfangsereignis EAn bzw. Ereignis En (z. B. EA1, EA2 bzw. E1, E2 usw.) - diese Umsetzung erfolgt vorliegend bevorzugt unter Bestimmung bestimmter Intensitätsunterschiede $\Delta I$ von Intensitäten I in Transmission T als Basis des spektroskopischen Messergebnisses MY in Bezug auf ein oder mehrere Grenzwerte lim1, lim2, die hier nur beispielhaft und konzeptionell vorab genannt sind. Eine weitere Erläuterung erfolgt in Bezug auf die Ansichten der FIG. 2 bis FIG. 4.

Des Weiteren ist ein zweites Steuermodul 500 vorgesehen, das ausgebildet ist, eine Warnung W und/oder einen Alarm A abzugeben, wenn das spektroskopische Messergebnis MY einen fehlerhaften Verlauf zu erkennen gibt bzw. derart gestaltet ist, dass Anlass für einen Alarm besteht - ein Fehler bzw. ein Alarm A würden einen sich verschlechternden bzw. alarmierenden Zustand des in der Maschine befindlichen Betriebsöls BÖ signalisieren.

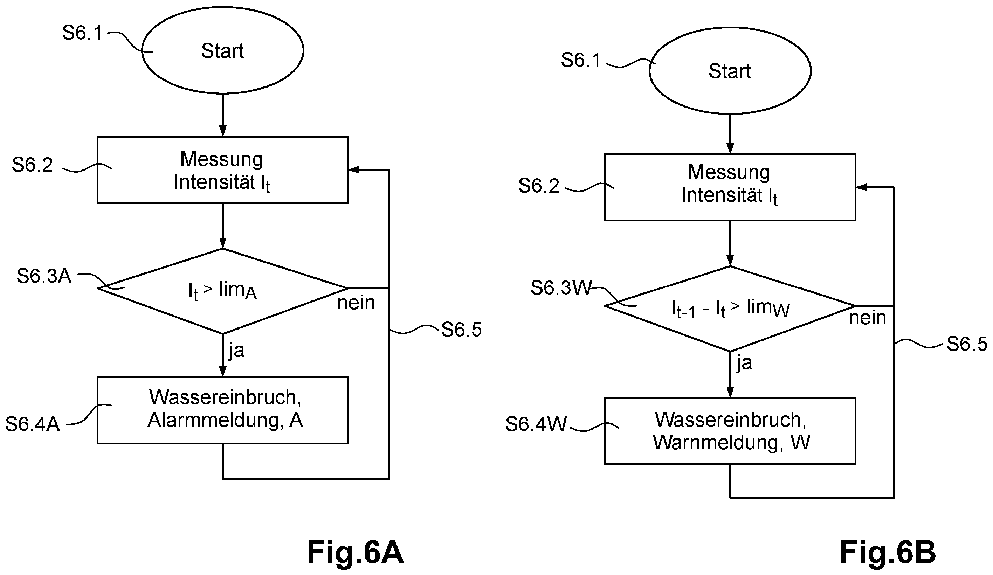

Vorliegend ist für das zweite Steuermodul 500 beispielhaft angegeben, dass dies im Falle eines Alarms A auf Grundlage eines Intensitätsunterschieds Delta_I im Sinne einer sprunghaften Änderungsamplitude $\Delta Y$ des Messsignals S in Bezug auf eine Alarmschwelle lim_A (FIG. 6A) angegeben wird und im Falle einer Warnung W auf Grundlage eines relativen Intensitätsunterschieds Delta_I im Sinne einer sprunghaften Änderungsamplitude $\Delta Y$ des Messsignals S in Bezug auf ein Fehlerband B bzw. einer diesem zugeordneten Warnschwelle lim_W angegeben wird (FIG. 6B).

Das erste Steuermodul 400 wird nachfolgend vor allem anhand der Ansichten der FIG. 2 bis FIG. 4 im Einzelnen beispielhaft mit bevorzugter Ausführungsbeispielen erläutert.

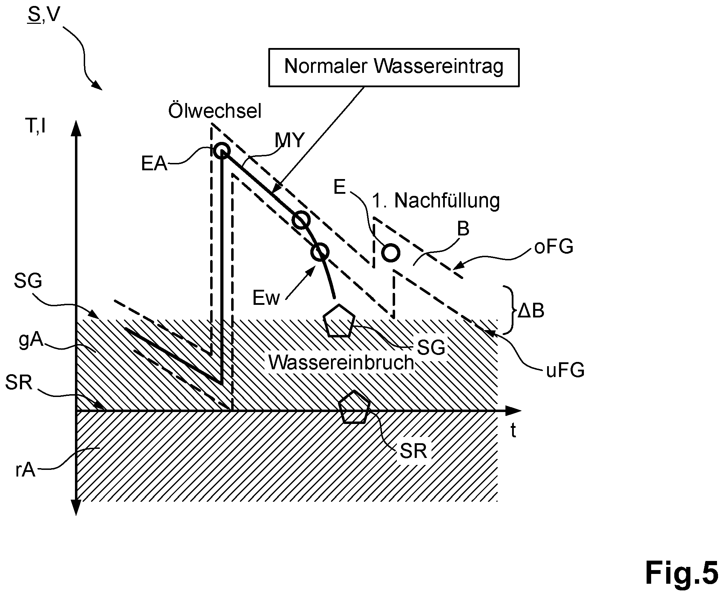

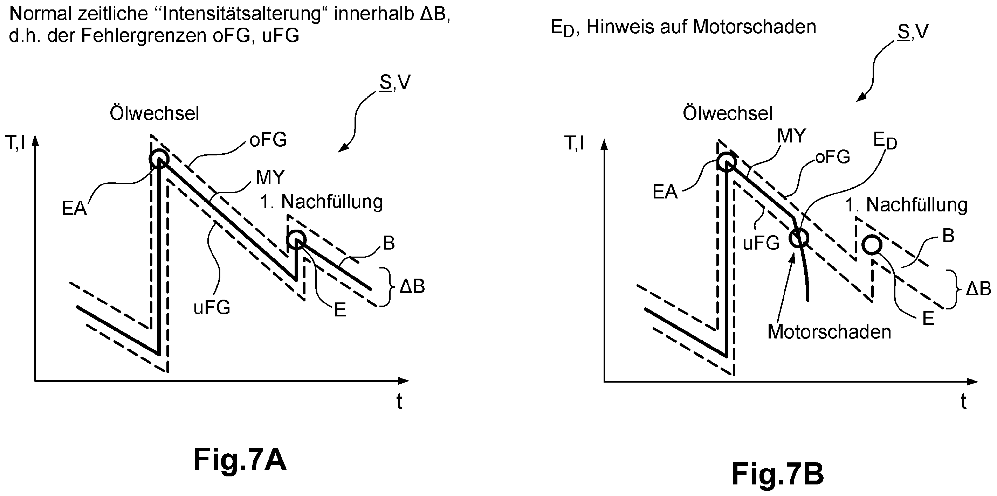

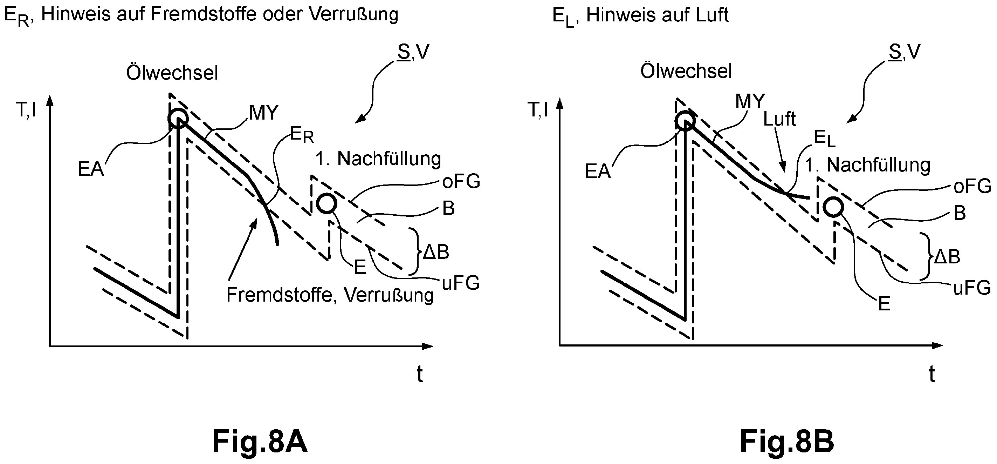

Das zweite Steuermodul 500 wird nachfolgend vor allem anhand des Ausführungsbeispiels der FIG. 5, der Ausführungsformen der FIG. 6A, FIG. 6B und der weiteren Ausführungsbeispiele der FIG.7A, FIG.7B und FIG.8A, FIG. 8B beispielhaft erläutert.

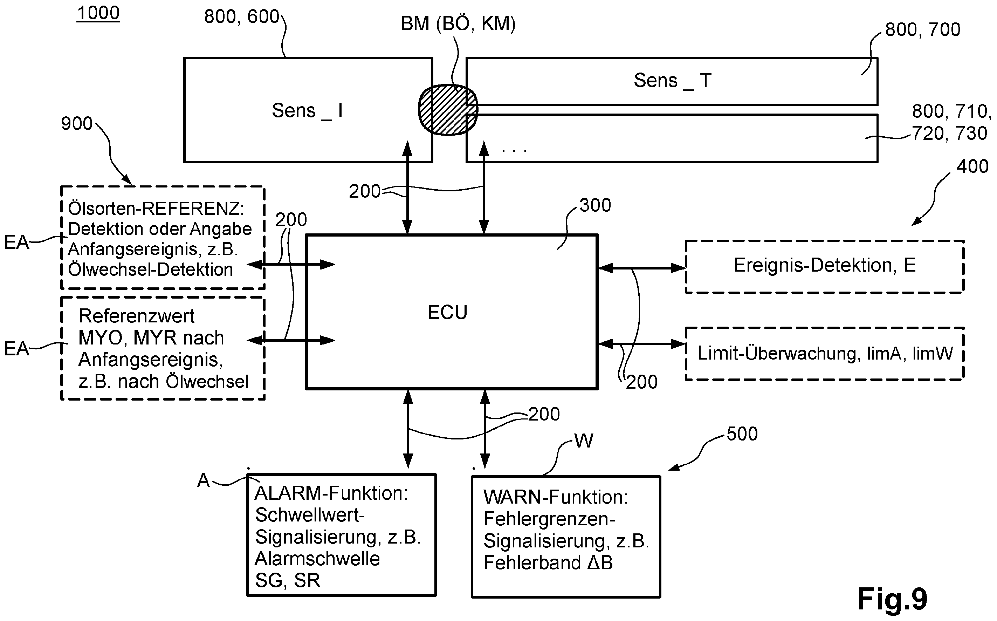

Die Steuereinheit 300 ist im Rahmen der Vorrichtung 1000 entsprechend im Detail erläutert in Bezug auf FIG. 9 und dort in einzelnen Aspekten schematisch gezeigt.

Im Folgenden wird anhand einer Berechnung beispielhaft gezeigt, wie bei der Transmissionsmessung für sich verschlechternde Werte für ein Betriebsöl BÖ --wie vorliegend Werte für Wasser im Betriebsöl BÖ -- verfahren wird.

FIG. 2 Ansicht (A) zeigt betreffend das Steuermodul 400 beispielhaft den Verlauf einer Intensität I über die Betriebszeit t, wobei die Intensität I in Transmission am Betriebsöl BÖ bestimmt ist. Erkennbar ist während des Verlaufs in der Betriebszeit t, dass ein Ölwechsel erfolgt ist - nach dem Ölwechsel liegt also ein Betriebsöl BÖ in der Maschine 100 vor, welches eine höhere Transmission T aufweist, so dass sich das spektroskopische Messergebnis MY vor dem Ölwechsel -zum Zeitpunkt t-1-- von dem nach dem Ölwechsel -zum Zeitpunkt t0-- durch eine sprunghafte Änderung in der Intensität I_t0 unterscheidet. Anders formuliert, liegt eine der sprunghaften Änderung (von Parametermesswert Y-1 auf Y0) zugeordnete sprunghafte Änderungsamplitude $\Delta Y0$ vor, die mit einer entsprechenden Änderungsamplitude des Messsignals S (d. h. S(Delta_I)) verbunden ist - insofern definiert dies hier ein Anfangsereignis EA.

Vorliegend ist dazu eine Intensität I_t0 sprunghaft erhöht zu einem Zeitpunkt t0 unmittelbar nach dem Ölwechsel im Vergleich zu einer Intensität I_t-1 zu einem Zeitpunkt t-1 vor dem Ölwechsel. Das entsprechende spektroskopische Messergebnis MY --zugeordnet der Intensität I_t-1 --ist vorliegend mit dem zugehörigen Parametermesswert Y-1 bezeichnet und das spektroskopische Messergebnis MY nach dem Ölwechsel --zugeordnet der Intensität I_t0-- ist vorliegend mit dem zugehörigen Parametermesswert Y0 bezeichnet. Beide spektroskopischen Messergebnisse MY, d. h. deren zugehörigen Parametermesswerte Y-1, Y0, sind vorliegend gestrichelt dargestellt.

Das Ereignis E --zuvor als Anfangsereignis EA bezeichnet-- eines Ölwechsels kann vorliegend als ein Anfangsereignis EA angesehen werden zu besagtem Zeitpunkt t0, um eine Messreferenz MY0 mit dem zugehörigen Parametermesswert Y0 in Bezug auf ein spektroskopisches Messergebnis MY anzugeben.

Dazu wird das spektroskopische Messergebnis MY gemäß dem Konzept der Erfindung über einen vorbestimmten auf das Anfangsereignis EA (in Form des Ölwechsels in Bezug auf das Betriebsöl BÖ) folgenden Referenzbetriebszeitraum R, d. h. zwischen einem Zeitpunkt t0 und tR als Anfangszeitpunkt und Endzeitpunkt des Referenzbetriebszeitraums R, wiederholt bestimmt. Die Bestimmung erfolgt beispielsweise mit 20-30 Wiederholungen über eine Betriebszeit t in Zeitschritten $\Delta$t.

Dazu werden in dem Referenzbetriebszeitraum R, d. h. zwischen den Zeitpunkten t0 und t_R, wiederholt bestimmte spektroskopische Messergebnisse MY angegeben. Es ist für einen bestimmten begrenzten und vergleichsweise zum Gesamtbetriebszeitraum der Maschine 100 gering gehaltenen Referenzbetriebszeitraum R nach dem Anfangsereignis EA (vorliegend in Form des Ölwechsels) davon auszugehen, dass sich die Eigenschaften des Betriebsöls BÖ nicht oder vernachlässigbar wenig ändern.

Es kann beispielweise eine wiederholte Bestimmung des spektroskopischen Messergebnisses MY dazu genutzt werden, für den Referenzbetriebszeitraum R aus den spektroskopischen Messergebnissen MY eine relevante Messreferenz MYR zu bestimmen - es ist davon auszugehen, dass diese mit dem Wert der Messreferenz Y0 übereinstimmt.

FIG. 2 Ansicht (B) zeigt dazu einen entsprechend geeigneten Verfahrensablauf zur Bestimmung der Messreferenz Y0. Nach der Angabe, dass das Anfangsereignis EA --vorliegend in Form eines Ölwechsels-- vorliegt, wird ein Ablauf zur wiederholten Bestimmung des spektroskopischen Messergebnisses MY unter Messung der Intensität I_t und zur Angabe des spektroskopischen Messergebnisses MY gestartet.

Nach dem Startschritt S 1.1 erfolgt also eine Messung der Intensität I_t am Betriebsöl BÖ im Messschritt S1.2; dies mit möglichst dichter oder jedenfalls geeigneter Zeitschrittsetzung von Zeitschritten $\Delta$t entsprechend einer sinnvollen Auflösung für den Referenzbetriebszeitraum R und unter wiederholter Bestimmung des spektroskopischen Messergebnisses MY am Betriebsöl BÖ.

Im Prüfschritt S1.3 wird geprüft, ob die im Nachfolgeschritt bestimmte Intensität I_t wesentlich größer als die vorherige im Schritt bestimmte Intensität I_t-1 ist. Ist dies nicht der Fall ("nein"), führt das Verfahren in einer Schleife S1.4 auf den Messschritt S1.2 zurück, d. h. im nächsten Zeitschritt erfolgt dann wiederum die Messung der Intensität I_t zur Bestimmung eines weiteren spektroskopischen Messergebnisses MY im Referenzbetriebszeitraum R.

Das spektroskopische Messergebnis MY wird also für eine Referenzanzahl von Wiederholungen wiederholt bestimmt unter Angabe einer Referenzanzahl von dem Referenzbetriebszeitraum R zugeordneten spektroskopischen Messergebnissen MY und der auf das Anfangsereignis EA in Bezug auf das Betriebsmittel BM folgende Referenzbetriebszeitraum R wird so festgelegt, dass die Referenzanzahl von spektroskopischen Messergebnissen MY im Referenzbetriebszeitraum R liegen.

Es hat sich gezeigt, dass das Ausführen dieser Schleife S1.4 etwa bis zu 10-mal oder 20-mal bei vergleichsweise geringen Zeitschritten $\Delta$t geeignet ist, eine verlässliche Messreferenz MY0 für das nach dem Ölwechsel --also nach dem Anfangsereignis EA-- in der Maschine 100 befindlichen Betriebsöl BÖ zu bestimmen.

Die relevante Messreferenz MYR kann beispielsweise unter anschließender Mittelwertbildung oder sonstiger Anwendung eines spektroskopischen oder numerischen Filters für die Messergebnisse MY im Referenzbetriebszeitraum R erfolgen. Es ist dazu zu verstehen, dass das Messergebnis MY grundsätzlich ein komplexes Messergebnis, beispielsweise eine einfache Intensität oder auch ein Intensitätsspektrum, sein kann, das frequenzaufgelöst bestimmt ist, für den Referenzbetriebszeitraum R.

Wenn die vorgenannten Zeitwerte oder Größenordnungen der Werte anfangs nicht bekannt sind, können geeignete Werte t, I_t und I_t-1, wie vormals gemessen, abgespeichert sein. Anhand von angelernten Werten kann eine Warnmeldung generiert werden, wann ein Ölwechsel stattzufinden hat.

Wenn die Werte zu Beginn bekannt sind oder angelernt wurden, speziell an verschiedenen Stellen eines Spektrums, so kann detektiert werden, ob immer die identische Ölsorte eingesetzt wird. Bei derselben Ölsorte müssten die Sprünge an allen Stellen des Spektrums relativ zueinander gleichartig ausfallen. Wird ein anderes Betriebsöl BÖ nachgefüllt, so erfolgt der Sprung nicht an allen Stellen im selben Verhältnis (z. B. wird ein anderes Betriebsöl BÖ eine andere Additivierung aufweisen).

Die Ölsorte kann anhand der spezifischen Relativänderungen auch beim Ölwechsel erkannt werden. Wird ein unbekanntes Betriebsöl BÖ nachgefüllt, so wird dies gemeldet und erst einmal werden die Grenzen des ursprünglich verwendeten Öls für Warnungen angenommen.

Um Fehlmessungen bei einer Ölnachfüllung zu vermeiden, kann vorteilhaft zudem Folgendes beachtet werden: Bei Ölnachfüllung sollten Anteile des Frischöls nicht über den Sensor geführt werden, sondern es sollte am Sensor vorbei zugeführt werden, so dass sich das bereits vorhandene Betriebsöl BÖ in der Ölwanne und das Frischöl gut durchmischen, bevor es den Sensor erreicht. Alternativ, wenn dies nicht möglich ist, sollten die Werte über ein Intervall betrachtet werden, bis diese einen eingeschwungenen Zustand erreichen (dann ist eine Vermischung erfolgt).

Gemäß dem Konzept der Erfindung kann also die vorgenannte relevante Messreferenz MYR (oder im einfachsten Fall auch die Messreferenz MY0, wie sie hier beispielhaft auf Grundlage einer Intensität I_t0 gezeigt ist), für eine bestimmte Sorte eines Betriebsöls BÖ festgelegt werden - insofern eine Kalibrierung ersetzen, wie sie bislang in der eingangs erläuterten Art im Stand der Technik erforderlich ist. Vorteilhaft erübrigt sich so eine separate Kalibrierung der Eigenschaften eines Betriebsöls BÖ; insbesondere erübrigt sich eine Entnahme des Betriebsöls BÖ aus der Maschine 100.

Vielmehr kann eine Bestimmung der Messreferenz MY0 --oder besser einer relevanten Messreferenz MYR-- für das Betriebsöl BÖ während des Betriebs, oder jedenfalls während sich das Betriebsöl BÖ in der Maschine 100 befindet, erfolgen. D. h. das zur Zustandsbestimmung vorgesehene Betriebsöl BÖ befindet sich bei der Zustandsbestimmung jedenfalls in der Maschine 100, wobei ein "Laufen" der Maschine 100 an sich nicht unbedingt erforderlich ist.

Es könnte insofern eine an die Maschine 100 gekoppelte Spektroskopieeinrichtung 600 mit eigener Energiequelle genutzt werden, ohne dass die Maschine 100 in Betrieb ist. Z. B. könnte die Spektroskopieeinrichtung 600 oder dgl. Analysevorrichtung mit einer eigenen Energiequelle ausgestattet sein, welche im Stillstand der Maschine 100 das in der Maschine 100 befindliche Betriebsöl BÖ analysiert. Wie erläutert, kann die Spektroskopieeinrichtung 600 Teil einer komplexeren Messeinrichtung 800 sein, die in der vorliegenden Ausführungsform auch eine Messeinheit 700 ausgebildet zur Bestimmung einer Temperatur aufweist.

Das Betriebsöl BÖ könnte z. B. über eine Vorpumpe durch die Maschine 100 --etwa durch eine Brennkraftmaschine oder einen sonstigen Motor-- gepumpt werden und davon unabhängig könnte ein Sensor der Spektroskopieeinrichtung 600 oder dgl. Analysevorrichtung betrieben werden; d. h. ggfs. auch unabhängig von einer ECU, 300 der Maschine 100. Dies hätte zudem einen Vorteil dahingehend, dass eine Zustandsbestimmung früh erfolgen kann; also vor einem Betrieb der Maschine 100 so könnte insbesondere eingedrungenes Wasser früher detektiert werden, da sich keine Seen von Wasser im Betriebsöl BÖ bilden, sondern Wasser und Betriebsöl BÖ dauernd vermischt wird. Der Sensor ist vorliegend ein Intensitätssensor Sens_I als Teil der Spektroskopieeinrichtung 600 und ein Temperatursensor Sens-T ist als Teil der Messeinheit 700 ausgebildet zur Bestimmung einer Temperatur.

BEISPIEL - Berechnung der Öleinfüllmenge am Beispiel von 100 1 Gesamtmenge (bei Ölwechsel als Beispiel eines Anfangsereignisses EA):

Y0 = Parametermesswert eines Messergebnisses MY --zugeordnet einer Intensität I_t0-- nach Ölwechsel (z. B. für Additive) zum Zeitpunkt t0;

Yn = Parametermesswert eines Messergebnisses MY --zugeordnet einer Intensität I_t-1-unmittelbar vor der Ölnachfüllung für Nachfüllung n = 1, 2, 3, ... zum Zeitpunkt t-1;

YnN = entsprechend Parametermesswert eines Messergebnisses MY unmittelbar nach der Ölnachfüllung für weitere Nachfüllung n = 1, 2, 3, ...;

$\Delta$YnN = Differenz des Parametermesswerts nach Ölnachfüllung; n = 1, 2, 3, ...;

Xn = Nachfüllmenge in Liter, im nachfolgenden Beispiel bei 100 1 Neuöl (nach Ölwechsel)

$\Delta YnN = YnN\text{-}Yn$ für Nachfüllung n = 1, 2, 3, ...; $\Delta$Y iSv Änderungsamplitude des Messsignals S

$$Xn = 100\ l * (\Delta YnN\ /\ Y0)$$

Selbstverständlich können die in diesem Beispiel genannten 100 Liter Neuöl (nach Ölwechsel) durch eine weitere Variable mit beliebiger Ölmenge ersetzt werden.

Bei der Darstellung der FIG. 3 wird anhand einer Berechnung beispielhaft schematisch gezeigt, wie bei einer Transmissionsmessung - d. h. für eine Messung einer Intensität I in Transmission T-- Isich verschlechternde Werte --wie beispielsweise für einen Anteil von Wasser im Betriebsöl BÖ-- erkennbar sind, und wie verfahren wird. Ein Ergebnis bei einer Reflexionsmessung -d. h. für eine Messung einer Intensität I in Reflexion R-- hat einen entsprechend gegensätzlichen, konkret komplementären, Verlauf von Werten (die normierten Werte einer Intensität I in Transmission T und Reflexion R addieren sich zu 1; sind insofern komplementär).

Der in FIG. 3 angegebene Verlauf des spektroskopischen Messergebnisses MY, basierend auf einer Intensität I über die Betriebszeit t, ergibt sich aus einem wiederholten Angeben des auswertbaren Messergebnisses MY derart, dass ein bestimmtes Zustandsmerkmal Z für das Betriebsöl BÖ signalisierbar ist. Das Zustandsmerkmal Z ergibt sich aus bestimmten Ereignissen E1, E2... En zu Zeitpunkten t1, t2 ... tn der Betriebszeit t, die hier beispielhaft erkennbar sind als erste Nachfüllung, zweite Nachfüllung bis zu einer beliebigen n-ten Nachfüllung für Betriebsöl BÖ.

Zusätzlich zu dem bereits anhand der Ansichten der FIG. 2 erläuterten Anfangsereignis EA (das hier insofern als erstes Anfangsereignis EA1 für einen ersten Ölwechsel bezeichnet ist) ist über den Verlauf der Betriebszeit t zum folgenden Zeitpunkt t02 ein weiteres Anfangsereignis EA2 in Form eines weiteren Ölwechsels erkennbar.

Für jedes der eine 1-te bis n-te Ölnachfüllung darstellendes weiteres Ereignis E1 bis En, lässt sich im Prinzip eine analoge Signatur erkennen, wie bei einem Ölwechsel, jedoch mit geringerer Änderungsamplitude $\Delta$Y eines sprunghaften Anstiegs, was durch die Differenzen der Parametermesswerte $\Delta$Y1n, $\Delta$Y2n, $\Delta$YnN, beim Verlauf des Messergebnisses MY in FIG. 3 erkennbar ist.

Nachfolgend wird beschrieben, wie sich ein Ölwechsel und eine Ölnachfüllung detektieren lassen.

1) Ein Ölwechsel kann folgendermaßen plausibilisiert werden:

Ein großer Sprung in den Messwerten des Messergebnisses MY zu Werten, die sich in Richtung Neuölzustand verändern, bedeutet "Ölwechsel", ein kleiner Sprung zu "besseren" Messwerten (in Richtung Neuölzustand) hin bedeutet "Öl wurde nachgefüllt".

Die Anzahl der Stunden der Betriebszeit t wird mit dem Datenlogger aufgezeichnet, auch über den Ölwechsel hinweg. Ein "großer" Sprung dient dann als Markierung, dass ein Ölwechsel stattgefunden hat und wird entsprechend im Datenlogger als Referenzmessung gekennzeichnet. Dazu geeignet sind all diejenigen Werte, die sich bei einem Ölwechsel stark verändern, da sich die Eigenschaften des Öls bei Alterung verändern, indem z. B. Additive abnehmen, bzw. 80 Säure- oder Basenwerte sich ändern, bzw. die Trübung sich erhöht und damit die Signalintensität an Referenzstellen abnimmt. In Bezug auf die Trübung werden als "Signalreferenz" Messstellen herangezogen, deren Wellenlängen nicht durch vorhandene Moleküle absorbiert werden, sondern rein ein Maß für die Opazität des Öls sind.

2) Bei einer Ölnachfüllung kann die Nachfüllmenge folgendermaßen plausibilisiert werden:

Die Abschätzung wird anhand von Messkriterien nachvollzogen, die messtechnisch überprüfbar sind.

a) Als Basis dienen der erste Messwert oder in kurzen Zeitintervallen über mehrere 90 Messungen hinweg gemittelte Messwerte (z. B. jede ½ Stunde eine Messung über 10 Messungen) unmittelbar nach dem Ölwechsel.

b) Zur Quantifizierung der Nachfüllmenge muss die im Motor vorhandene Gesamtölmenge bekannt sein.

c) Bei einer Nachfüllung entsteht einer "kleiner" Sprung in den Messwerten. Dieser "kleine" Sprung wird ins Verhältnis gesetzt zu einem "großen" Sprung (bei einem Ölwechsel), woraus die Nachfüllmenge errechnet wird.

d) Optional kann man die Nachfüllmenge mit einem elektrischen Ölstandssensor (bei Motorstillstand und einer definierten Temperatur) plausibilisieren, falls vorhanden.

Geeignet für diese Methode sind gemessene Stoffe, die sich während des Motorbetriebs nicht durch äußere Veränderungen beeinflussen lassen und deren Werte sich proportional mit der nachgefüllten Menge verhalten. Geeignet sind typischerweise:

- Additive: Diese eignen sich, da sie sich nur beim Nachfüllen von Frischöl wieder erhöhen unter der Voraussetzung, dass derselbe Öltyp verwendet wird.
- Sulfate: Dies unter der Voraussetzung, dass der Motor mit schwefelarmem Kraftstoff nach Norm (z. B. DIN 110 EN 590) betrieben wird. Kommt schwefelhaltiger Kraftstoff zum Einsatz, können Sulfate zur Bewertung nicht herangezogen werden.

Eher weniger geeignet oder ungeeignet sind Aspekte wie z. B.:

- Wasser: Es entsteht bei der Verbrennung und kann noch im geringen Umfang durch hohe Luftfeuchtigkeit im Zylinder bei der Verbrennung als Blow-By an den Kolbenringen vorbei ins Betriebsöl BÖ A gelangen.

Zusätzlich erhöhen viele Motorstarts und kurze Motorlaufzeiten den Wassergehalt im Öl.

- Basenzahl, Säurezahl: Diese wird -wie zuvor erläutert-- durch zusätzliche chemische Reaktionen bei der Verbrennung beeinflusst, abhängig vom Motorbetrieb (vor allem dem Verbrennungsluftverhältnis), Kraftstoffqualität, Motorzustand (z. B. Alterung der Kolbenringe), und entsprechend ändern sich diese Werte. Zusätzlich spielt die Betriebstemperatur eine Rolle und abhängig von der Umgebung die atmosphärische Zusammensetzung.

- Oxidation, Nitration: Diese sind zu sehr abhängig vom Motorbetrieb, von der Kraftstoffqualität, Motorzustand und den Umgebungsbedingungen.

Weiterhin geeignet ist aber insbesondere eine reine Intensitätsmessung in einem Infrarotmessbereich, in dem optisch aktiven Substanzen nicht vorhanden sind bzw. nicht vorhanden sein müssen. Die Intensitätsabnahme erfolgt dann rein aufgrund der Messung einer Trübung des Betriebsöls BÖ, wie dies beispielsweise im Graphen für den Verlauf V des auswertbaren Messsignals S in der FIG. 3 erkennbar ist; dies unter Berücksichtigung der Referenzmessung im Referenz-Betriebszeitraum R wie dies anhand der Ansichten der FIG. 2 gezeigt und erläutert ist.

Eine Ölnachfüllung im Bereich des Ereignisses E1... En ist erkennbar über einen Wert des Anstiegs der oberhalb eines zweiten Grenzwert lim2 liegt, für die gemessene Intensität als Basis für das spektroskopische Messergebnis MY, wobei dieser zweite Grenzwert geringer ist als der Grenzwert lim2 bei einem Anfangsereignis EA1, EA2 eines Ölwechsels.

Dies erlaubt zum einen die Bestimmung eines Ölwechsels als Anfangsereignis EA1, EA2 oder als einfaches Ereignis E. Dies erlaubt des Weiteren die Bestimmung einer Ölnachfüllung als eines oder mehrere der Ereignisse E1 bis En. Es erlaubt über die Unterscheidung eines Intensitätsunterschieds Delta_I im Sinne einer sprunghaften Änderungs- amplitude $\Delta Y$ des Messsignals S in Bezug auf den Amplitudenwert lim2 --als kleinerer Amplitudenwert lim2 gegenüber dem größeren Amplitudenwert lim1-- die Unterscheidung zwischen einem Ölwechsel (zugeordnet einer größeren Änderungsamplitude $\Delta Y$ des Messsignals S über dem Amplitudenwert lim1) im Vergleich zu einer Ölnachfüllung (mit kleinerer Änderungsamplitude $\Delta Y$ des Messsignals S über dem kleineren Amplitudenwert lim2).

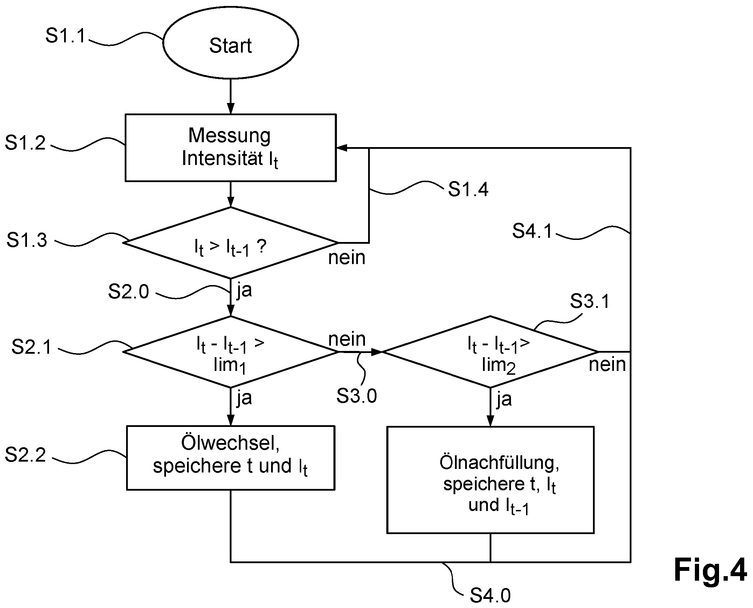

Ein entsprechendes Ablaufdiagramm eines Verfahrens zur Zustandsbestimmung des in der Maschine 100 befindlichen Betriebsöls BÖ --ausführbar in dem in FIG. 1 gezeigten ersten Steuermodul 400-- ist in FIG. 4 dargestellt; dabei erfolgt eine Ölwechseldetektion oder dergleichen Detektion eines Anfangsereignisses EA und weiteren Ereignisses E.

Die zuvor erläuterte Bestimmung der Messreferenz MY0 nach einem Anfangsereignis EA in Form eines ersten Ölwechsels (erstes Anfangsereignis EA1 oder zweites Anfangsereignis EA2) ist ein Teil dieses Verfahrensablaufs in dem Ausführungsbeispiel der FIG. 4. Betreffend die Schritte S1. 1 bis S1.4 im Verfahrensablauf wird auf den Verfahrensablauf verwiesen, wie er in dem Ausführungsbeispiel der Ansichten der FIG. 2, insbesondere gemäß der Ansicht (B), dargestellt ist.

Für den Fall, dass im Schritt S1.3 darauf zu erkennen ist, dass die Änderungsamplitude in der Intensität I_t im Vergleich zur Intensität I_t-1 im vorhergehenden Schritt angestiegen ist --d. h. in dem Fall die Transmission T zugenommen hat-- wird im Schritt S2.0 dazu übergegangen ("ja"), die zuvor erläuterten Grenzwerte lim1, lim2 als größerer und kleinerer Amplitudenwert abzufragen.

So wird im Schritt S2.1 abgefragt, ob die Nachfolgeintensität I_t, die zuvor bestimmte Intensität I_t-1 um einen ersten Grenzwert lim1 übersteigt. Konkret wird abgefragt, ob die Änderungsamplitude $\Delta Y$ bzw. S (I_t - I_t-1) des Messsignals S den ersten Grenzwert lim1 als größeren Amplitudenwert übersteigt. Ist dies der Fall ("ja"), wird im Schritt S2.2 auf einen Ölwechsel erkannt und der entsprechende Betriebszeitpunkt t und die Intensität I_t dazu -bzw. die der Messwert MY als Messreferenz MY0 (vgl. Ausführungsbeispiel in den Ansichten der FIG. 2) abgespeichert.

Wird dagegen die Abfrage verneint ("nein") im Schritt S3.0, wird im Schritt S3.1 geprüft, ob die nachfolgende Intensität I_t zum Zeitpunkt t die zuvor gemessene Intensität I_t-1 um einen anderen, zweiten Grenzwert lim2 übersteigt. Konkret wird abgefragt, ob die Änderungsamplitude $\Delta Y$ bzw. S (I_t - I_t-1) des Messsignals S den zweiten Grenzwert lim2 als kleineren Amplitudenwert übersteigt. In diesem Fall der Messung als Transmission T ist der zweite Grenzwert lim2 kleiner ist als der erste Grenzwert lim1.

Diese Situation trifft beispielsweise zu für die Betriebszeitpunkte t1, t2 und tn mit dort vorliegenden spektroskopischen Messergebnissen MY; d. h. jeweils nach der Ölnachfüllung entsprechend der Parametermesswerte Y1, Y1N bzw. Y2, Y2N bzw. Yn, YnN usw. Überschreitet also eine dort gemessene Intensitätsänderung $\Delta$I1, $\Delta$I2, $\Delta$In bzw. die entsprechende sprunghafte Änderung des Messergebnisses MY bzw. die Differenz der Parametermesswerte $\Delta$Y1N, $\Delta$Y2N, $\Delta$YnN im Sinne einer Änderungsamplitude $\Delta Y$ bzw. S (I_t - I_t-1) des Messsignals S den zweiten Grenzwert lim2, wird auf eine Ölnachfüllung geschlossen und der entsprechende Betriebszeitpunkt t mit Intensitätswerten I_t und I_t-1 bzw. die entsprechende Amplitude des Messsignals S abgespeichert.

Danach wird der Ablauf mit einem weiteren Schritt S4.0 zum nächsten Zeitschritt geführt, in dem eine weitere Messung der Intensität I_t+1 erfolgt. Dies ist auch der Fall, wenn die Abfrage im Schritt S3.1 ("nein") nicht positiv beantwortet wird, so dass der Schritt S4.1 das Verfahren an den Anfang zum Messschritt S1.2 zurückführt.

Die Darstellung in FIG. 5 veranschaulicht eine Detektion eines Wassereinbruchs im Betriebsöl BÖ. In FIG. 5 ist zunächst am Beispiel der Messung der Transmission T erkennbar, dass das Signal S eines spektroskopischen Messergebnisses MY mit zunehmender Verschmutzung des Betriebsöls BÖ in der Gesamtentwicklung abnimmt. Bei einem Ölwechsel oder Ölverdünnung wäre die Verschmutzung zunehmend geringer und es gäbe einen Signalsprung zu hoher Transmission wie dies anhand von FIG. 4 erläutert ist, oder jedenfalls eine zunehmende Signaltendenz für das spektroskopische Messergebnis MY. Im Übrigen sind auch in FIG. 5 ein Anfangsereignis EA eines Ölwechsels und ein (prognostiziertes) Ereignis E einer Ölnachfüllung beispielhaft erkennbar.

Dazu zeigt FIG. 5 einen exemplarischen Signalverlauf V für das Signal S analog zur FIG. 2 Ansicht (A) und FIG. 3, wobei zudem ein Wassereinbruch im Signalverlauf als Ereignis E_W erkennbar ist und der Signalverlauf eine untere Fehlergrenze uFG eines Trendbandes B mit oberer und unterer Fehlergrenze oFG, uFG unterschreitet. Dies führt zur Angabe eines Warnhinweises W.

Der Kühlwassereinbruch wird vorliegend durch eine Transmissionsmessung T detektiert, bei der an einer Infrarotfrequenz für Wasser und ggfs. auch an der Infrarotfrequenz von z. B. Natrium (Kühlmittelzusatz) gemessen wird. Ähnlich würde eine Reflexionsmessung möglich sein, mit qualitativ analogen Maßgaben, wie hier am Beispiel einer Transmissionsmessung T erläutert ist. Die Messung wird relativ auf die Zeit t bezogen, da das Betriebsöl BÖ Wasser langsam durch die Umgebungsluft und durch das bei einer Verbrennung entstehende Wasser, via "Blow-By" an den Kolbenringen vorbei, aufnimmt.

Beim Beispiel des Wassereinbruchs mittels Intensitätsmessung (ggfs. ohne die Option eines Trendbandes B oder einer unteren oder oberen Fehlergrenze uFG, oFG) gem. FIG. 5 wird zudem vom spektroskopischen Messergebnis Y schließlich ein erster unterer Schwellwert SG unterschritten; es erfolgt Gelbalarm gA. Wird der zweite untere Schwellwert SR unterschritten, erfolgt Rotalarm rA. Bei jedem Alarmtyp und je nach Anwendung können entsprechende Maßnahmen, bis zur automatisierten Motorabstellung, eingeleitet werden.

Desgleichen kann auch ein Absorptionsspektrum herangezogen werden, das quasi invertiert verläuft. Das Signal nähme dann durch eine zunehmende Verschmutzung als Funktion der Zeit in der Gesamtentwicklung zu. Bei einem Ölwechsel erfolgt der Signalsprung nach unten, da durch die verringerte Verschmutzung die Absorption sehr klein ist; entsprechend würden die Alarme dann angepasst werden.

Dazu zeigt generell FIG. 6A ein Ablaufdiagramm für eine erste Ausführungsform eines Ablaufs eines Verfahrens zur Angabe eines Alarmhinweises und FIG. 6B zeigt ein Ablaufdiagramm für eine erste Ausführungsform eines Ablaufs eines Verfahrens zur Angabe eines Warnhinweises. Eine entsprechende Steuerung umfasst ein Modul 500 (wie in FIG. 1 gezeigt und erläutert) mittels dem ein Warn- und/oder Alarm-Zustand für ein in einer Maschine befindliches Betriebsöl BÖ verlässlicher detektierbar ist.

Nach einem ersten Startschritt S6.1 wird mit der Spektroskopieeinrichtung 600 mit wenigstens dem Intensitätssensor Sens_I eine Intensität I am Betriebsöl BÖ gemessen im Schritt S6.2. Im Prüfschritt S6.3A bzw. S6.3W wird diese auf eine Bedingung geprüft. Vorliegend ist für das zweite Steuermodul 500 beispielhaft angegeben, dass dies auf Grundlage eines Intensitätsunterschieds in Bezug auf ein Fehlerband bzw. eines Intensitätsunterschieds in Bezug auf einer Alarmschwelle angegeben wird; d. h. der Prüfschritt S6.2A prüft auf die Intensität I_t absolut in Bezug auf die Alarmschwelle limA, d. h. die Alarmschwelle Gelbalarm SG oder Rotalarm SR -in dem vorliegend beschriebenen Ausführungsbeispiel der erste und zweite untere Schwellwert SG, SR-- wohingegen der Prüfschritt S6.2B die Intensität I_t relativ zu einer Abweichung in Bezug auf eine Fehlerabweichung ΔB des Trendbandes B prüft. Die Fehlerabweichung ΔB ist im Wesentlichen durch die vorgenannten unteren oder oberen Fehlergrenze uFG, oFG vorgegeben.

Erkennbar ist, dass ein Alarmhinweis A im Schritt S6.4A im Grunde erst bei Überschreiten eines Schwellwertes lim_A durch einen Absolutwert der Intensität I_t bzw. des damit verbundenen Messsignals S erfolgt, wohingegen ein Warnhinweis im Schritt S6.4W bereits bei Überschreiten einer Warnschwelle lim_W, nach Vorgabe der Fehlergrenzen uFG, oFG, durch eine Differenz oder dgl. Relativwert der Intensität I_t-1 - I_t bzw. der damit verbundenen Differenz der Messsignale S erfolgt.

Das Verfahren wird als eine Schleife ausgeführt mit einem Rückkoppelschritt S6.5 zum Messschritt S6.2.

FIG. 7A, FIG. 7B und FIG. 8A, FIG. 8B zeigen analog zu FIG. 5 weitere exemplarische Signalverläufe, wobei zudem ein weiteres Ereignis im Signalverlauf erkennbar ist und der Signalverlauf eine untere Fehlergrenze uFG eines Trendbandes B unterschreitet. Dies führt zur Angabe eines Warnhinweises.

Zunächst zeigt FIG. 7A für einen Normalbetrieb anhand einer Transmissionsmessung T die an sich bekannten Trends einer Ölverschmutzung - demnach können optional obere und untere Fehlergrenzen oFG, uFG eines Trendbandes B gesetzt werden, wie dies bereits anhand FIG. 5 erläutert ist. Werden diese obere und/oder untere Fehlergrenze oFG, uFG als Fehlergrenzenverlauf unter- oder überschritten kann der Betreiber darauf hingewiesen werden (z. B. über ein Display), dass etwas an der Maschine 100 od. dgl. Gerät außergewöhnlich abläuft und diese zu überprüfen ist. Zusätzlich oder alternativ zu Fehlergrenzen uFG, oFG kann auch ein Gradientenverlauf herangezogen werden.

Ein Überschreiten eines Trendbandes B kann bereits zu einem frühen Hinweissignal führen, das dazu dient, bei Überschreiten einer oberen oder unteren Fehlergrenze oFG, uFG des Trendbandes B eine frühe Warnung W auf eine Anomalität des Öl-Zustands zu liefern. Anders ausgedrückt, zeigt sich mit Vorteil, dass ein solcher früher Hinweis deutlich vor einem Alarm A (etwa einem Gelb-Alarm gA oder Rot-Alarm rA mit festen Schwellwerten) erfolgen kann, da ein Alarm wiederum nur anhand statischer Maßgaben (und damit ggfs. zu spät) erfolgt.

In einem Alarmzustand des Betriebsöls BÖ kann ein Motor oder ein technisches System oder dergleichen Maschine 100 bereits in Gefahr sein infolge eines Öl-Zustands, der sich bereits zu stark verschlechtert hat.

Es zeigt sich, dass eine Basis für das Trendband B die Beseitigung von Mess-, Produktions- und Temperaturtoleranzen im Betriebsöl BÖ ist - diese wird gewährleistet durch die erfindungsgemäße Bestimmung der Messreferenz aus den spektroskopischen Messergebnissen.

So wird die Produktionstoleranz bereits beseitigt durch die Referenzintensität; denn das Betriebsölwird BÖ als jenes angenommen, was im Motor befindlich ist.

Die Temperaturtoleranz kann mitgemessen werden und somit berücksichtigt werden. Entsprechend kann eine Temperatur im Rahmen einer komplexen Messeinrichtung 800 mit der in FIG. 1 und FIG. 9 gezeigten und erläuterten Messeinheit 700, ausgebildet zur Bestimmung einer Temperatur, die hier einen Temperatursensor Sens_T aufweist, mitgemessen werden. Es können auch weitere Toleranzen, wie Wassereintrag oder dergleichen etwa durch Erfahrungswerte oder dergleichen angelernt werden. Es können im Rahmen der komplexen Messeinrichtung 800 auch weitere Messeinheiten vorgesehen sein.

Auch dient ein vorgenanntes Trendband selbst insbesondere dazu, eben jene Toleranzen zu akzeptieren. Im vorliegenden Fall sind zudem eben jene Toleranzen beseitigt, d. h. die Öl-Zustandsbestimmung und Überwachung ist deutlich genauer und das Toleranzband ist somit in besonders genauer Weise ausgebildet, frühe Öl-Verschlechterungen anzeigbar zu machen.

In FIG. 7B ist die Detektion von Dieselkraftstoff im Betriebsöl BÖ veranschaulicht. Dies ist als ein weiteres Ereignis E_D im Signalverlauf erkennbar und der Signalverlauf unterschreitet eine untere Fehlergrenze uFG eines Trendbandes B.

Die beschriebene Methode mittels Fehlergrenzen uFg, oFG des Trendbandes B kann zusätzlich oder alternativ analog angewandt werden mit einer Gradientenbestimmung.

Ein Kraftstoffeintrag kann beispielsweise über einen schnellen Abfall von Öladditiven wie u. a. Zink, Barium, Bor, Calcium, Magnesium oder Phosphor - abhängig von der Grundadditivierung des Öls - 250 detektiert werden.

Die Signaländerungen bei einem sich anbahnenden Motorschaden sind von folgender Art:

- Schnelle Signaländerung durch Verdünnung des Öls,
- Schnelle Signaländerung durch Temperaturänderung des Öls, falls der Temperaturunterschied zwischen Wasser oder Kraftstoff im Vergleich zum Motoröl relativ groß ist.

Der Signaltrend erfolgt durch Gradientenbestimmung. In der Regel ist der Abnahmetrend der Additive anhand von Aufzeichnungen über eine längere Zeit bekannt, da diese in modernen Geräten über Tage/Wochen gespeichert werden. Verändert sich der Gradient innerhalb eines festgelegten Zeitintervall stark von den bisherigen Aufzeichnungen, wird alarmiert.

FIG. 8A veranschaulicht anhand eines Beispiels einer Transmissionsmessung, die Detektion von Verschmutzung und/oder Ruß und/oder Stoffen im Betriebsöl BÖ, die nicht im Sensorbereich liegen. Dies ist als ein weiteres Ereignis E_R im Signalverlauf erkennbar und der Signalverlauf unterschreitet eine untere Fehlergrenze uFG eines Trendbandes B.

Es ist eine breitbandige Messung anstelle nur an einem Band (Messparameter) möglich, da Ruß generell infrarotaktiv ist.

Alternativ können auch schmalbandige Bereiche herangezogen werden, die nicht im Bereich der zu untersuchenden Substanzen (Wasser, Nitrate, Sulfate, etc.) liegen, sondern nur auf Ruß (infrarotaktiv) und Verschmutzung (Transmissions- bzw. Absorptionsintensität) reagieren.

Wenn schmalbandige Spetrumsbänder eines Spektrums der Messstrahlung "außerhalb der gezielt untersuchten Substanzen (wie oben schon genannt)" keine Veränderung zeigen oder nur eine geringere, so liegt ein Eintrag einer unbekannten Stoffart vor. Bei starker Trübung jedoch, d. h. bei starkem Pegelabfall der Intensität I bei Transmissionsmessung T, ist es meist Ruß, da dieser auf viele Wellenlängen im Spektrum der transmittierten Messstrahlung eine Auswirkung hat.

Auch die Signalintensität ist ein Maß für die Verschmutzung des Öls. Analog zur Vorgehensweise bei Ölalterung kann die Signalintensität als weiterer Messindikator herangezogen werden. Nimmt die Intensität gemäß Vorgaben innerhalb der Fehlergrenzen oFG, uFG ab oder fällt ein Gradient relativ gleichmäßig ab, dann handelt es sich um eine normale Alterung des Öls. Nehmen ein oder mehrere Parameter sehr schnell ab, dann handelt es sich eher um ein Motorproblem. Es sollte berücksichtigt werden, dass bei Ölnachfüllung die Opazität des Öls abnimmt.

FIG. 8B veranschaulicht anhand eines Beispiels einer Transmissionsmessung, einen Hinweis auf Luft. Dies ist als ein weiteres Ereignis E_L im Signalverlauf erkennbar ist und der Signalverlauf überschreitet eine obere Fehlergrenze oFG eines Trendbandes B.

Oftmals ist Luft im Öl -dies bewirkt einen Signalsprung an allen Messstellen. Ist der Sprung nur bei einer Messstelle zu beobachten, dann wird ein Fremdstoff eingetragen. Bei Luft ist die Transparenz des Öls höher und die Signalintensität nimmt bei der Transmissionsmessung zu. Bei Fremdstoffen nimmt die Trübung des Öls zu und aufgrund von Brechung nimmt die Signalintensität ab.

FIG. 9 zeigt konkret schematisch ein Steuer- und Messsystem 1000 im Detail mit der aufgezeigten Funktionalität zur Bestimmung eines Ölzustands gemäß einer bevorzugten Weiterbildung.

Ausgehend von FIG. 1 ist in FIG. 9 in einer Übersicht eine bevorzugte Ausführungsform einer Vorrichtung 1000 gemäß dem Konzept der Erfindung gezeigt; hier nämlich die Steuereinheit ECU, 300 und im Rahmen einer komplexen Messeinrichtung 800 eine Spektroskopieeinrichtung 600 mit einem Intensitätssensor Sens_I und eine Messeinheit 700 ausgebildet zur Bestimmung einer Temperatur, die hier einen Temperatursensor Sens_T aufweist und ggfs. eine oder mehrere weitere Messeinheiten 710, 720, 730 jeweils zur Bestimmung einer Viskosität, Trübung oder Dichte des Betriebsmittels BM.

Die Vorrichtung 1000 weist eine entsprechende Einrichtung 200 zur Signalisierung bzw. zum Überwachen oder dgl. zur Angabe bzw. Anzeige eines Zustandes eines in der Maschine befindlichen Betriebsöls BÖ auf; die Kommunikation zwischen Steuereinheit ECU, 300 und den weiteren Komponenten der Vorrichtung 1000 verläuft bidirektional mittels der Einrichtung 200 zur Signalisierung bzw. zum Überwachen.

Die Vorrichtung 1000 ist ausgebildet zur Ausführung des Verfahrens zur Zustandsbestimmung eines in einer Maschine 100 befindlichen Betriebsmittels BM, wie insbesondere aber nicht ausschließlich eine Betriebsöls BÖ, insbesondere mit einer Ölwechseldetektion oder dergleichen Detektion eines Anfangsereignisses EA oder eines Ereignisses E.

Die Vorrichtung 1000 ist insbesondere ausgebildet mit einer (hier nicht gezeigten) Zuführung von Betriebsöls BÖ der Maschine zu einer an die Maschine gekoppelten Spektroskopieeinrichtung 600 mit dem Intensitätssensor Sens_I. Außerdem ist die Spektroskopieeinrichtung 600 mit dem Intensitätssensor Sens_I ausgebildet zum Durchführen einer spektroskopischen Messanalyse mittels einer Messstrahlung der Spektroskopieeinrichtung und zum Bestimmen eines spektroskopischen Messergebnisses MY basierend auf der spektroskopischen Messanalyse.

Die Vorrichtung 1000 ist außerdem mit einem z. B. in der Steuereinheit 300 untergebrachten Rechenmodul ausgestattet, das es erlaubt das spektroskopische Messergebnis MY in Relation zu einer Messreferenz MY0 bzw. der relevanten Messreferenz MYR, derart zu setzen, dass aus der Relation ein auswertbares Messsignal S angegeben wird. So kann das auswertbare Messsignal S über eine Betriebszeit t der Maschine 100 wiederholt angegeben werden, derart, dass ein bestimmtes Zustandsmerkmal Z für das Betriebsöl BÖ signalisierbar ist.

Des Weiteren ist ein Referenzmodul 900 vorgesehen, das ausgebildet ist -wie anhand der Ansichten der FIG. 2 erläutert-- das spektroskopische Messergebnis MY über einen vorbestimmten, auf ein Anfangsereignis EA in Bezug auf das Betriebsöl BÖ folgenden Referenzbetriebszeitraum R wiederholt zu bestimmen unter Angabe von dem Referenzbetriebszeitraum R zugeordneten spektroskopischen Messergebnissen MY, und aus den spektroskopischen Messergebnissen die Messreferenz MY0, MYR zu bestimmen. Damit kann beispielsweise auch eine Ölsorte bestimmt werden.

Das Referenzmodul 900 ist zudem weiter ausgebildet, das Anfangsereignis EA in Bezug auf das Betriebsöl als ein zum Bestimmen der insbesondere relevanten Messreferenz MY0, MYR bestimmtes oder geeignetes Anfangsereignis EAn in Bezug auf das Betriebsöl BÖ oder dgl. Betriebsmittel BM auszuwählen; insbesondere in Form eines Ölwechsels, ggfs. aber auch einer Ölnachfüllung. Das Referenzmodul 900 ist ausgebildet, das Anfangsereignis EA in Bezug auf das Betriebsöl BÖ festzustellen, derart, dass geprüft wird, dass das Messsignal S für ein Ereigniszeitfenster eine sprunghafte Änderung im Sinne einer Änderungsamplitude S(Delta_I) des Messsignals S aufweist.

Die Vorrichtung 1000 weist auch ein erstes Steuermodul 400 auf, das ausgebildet ist, das spektroskopische Messergebnis MY auszuwerten im Hinblick auf die Bestimmung einer insbesondere relevanten Messreferenz MY0, MYR und/oder ein Ereignis E - diese Umsetzung erfolgt vorliegend bevorzugt unter Bestimmung bestimmter Intensitätsunterschiede ΔI als Basis des spektroskopischen Messergebnisses MY in Bezug auf ein oder mehrere Grenzwerte lim1, lim2.

Das erste Steuermodul 400 ist insbesondere ausgebildet, eine der sprunghaften Änderung zugeordnete sprunghafte Änderungsamplitude S(Delta_I) des Messsignals S zu identifizieren, wenn diese einen ersten Ereignisschwellwert lim1 überschreitet bzw. passiert, sodass damit das Anfangsereignis oder ein anderes Ereignis als ein Ölwechsel identifiziert werden kann. Das erste Steuermodul 400 ist insbesondere ausgebildet, eine der sprunghaften Änderung zugeordnete sprunghafte Änderungsamplitude zu identifizieren, wenn diese einen zweiten Ereignisschwellwert lim2 überschreitet bzw. passiert, sodass damit das Anfangsereignis oder ein anderes Ereignis als eine Ölnachfüllung identifiziert werden kann. Der erste Ereignis-Schwellwert lim1 überschreitet den zweiten Ereignis-Schwellwert lim2 im Fall einer Transmissionsmessung.

Die Vorrichtung bzw. Steuer- und Messvorrichtung 1000 weist auch ein zweites Steuermodul 500 auf, das ausgebildet ist, eine Warnung W und/einen ein Alarm A abzugeben, wenn das spektroskopische Messergebnis MY einen fehlerhaften Verlauf zur erkennen gibt bzw. derart gestaltet ist, dass Anlass für einen Alarm besteht - ein Fehler bzw. ein Alarm A würde einen sich verschlechternden bzw. alarmierenden Zustand des in der Maschine 100 befindlichen Betriebsöls signalisieren.

Vorliegend ist für das zweite Steuermodul 500 beispielhaft angegeben, dass dies im Falle eines Alarms A auf Grundlage eines Intensitätsunterschieds Delta_I bzw. einer zugeordneten Änderungsamplitude S(Delta_I) des Messsignals S in Bezug auf eine Alarmschwelle limA (gezeigt in FIG. 6A) angegeben wird und im Falle einer Warnung W auf Grundlage eines relativen Intensitätsunterschieds Delta_I bzw. einer zugeordneten Änderungsamplitude S(Delta_I) des Messsignals S in Bezug auf ein Trendband B bzw. dessen Fehlerabweichung ΔB angegeben wird (FIG. 6B). Insbesondere ist das zweite Steuermodul 500 ausgebildet zum wiederholt angegebenen auswertbaren Messsignal MY über die Betriebszeit t eine obere und/oder untere Fehlergrenze oFG, uFG oFg, uFG anzugeben; insbesondere über einen Verlauf des auswertbaren Messsignals MY über die Betriebszeit t die obere und/oder untere Fehlergrenze oFG, uFG als Fehlergrenzenverlauf anzugeben, die einen zu bevorzugenden Verlauf V des auswertbaren Messsignals S vorgeben.

BEZUGSZEICHENLISTE

| | |
|---|---|
| 100 | Maschine |
| 200 | Einrichtung zur Signalisierung bzw. zum Überwachen oder dgl. zur Angabe bzw. Anzeige eines Zustandes eines in der Maschine befindlichen Betriebsöls |

| 300 | Steuereinheit |
| 400 | Erstes Steuermodul |
| 500 | Zweites Steuermodul |
| 600 | Spektroskopieeinrichtung |
| 700 | Temperaturmesseinheit |
| 710, 720, 730 | weitere Messeinheit jeweils zur Bestimmung einer Viskosität, Trübung oder Dichte des Betriebsmittels BM |
| 800 | Messeinrichtung |
| 900 | Referenzmodul |
| 1000 | Vorrichtung |
| Sens_I | Intensitätssensor |
| uFG, oFG | untere, obere Fehlergrenze |
| I_t-1 - I_t | Intensität |
| W | Warnung |
| A | Alarm A |
| gA, rA | Gelb-Alarm, Rot-Alarm |
| SG | erster unterer Schwellwert, Alarmschwelle Gelbalarm |
| SR | zweiter unterer Schwellwert, Alarmschwelle Rotalarm |
| lim_A lim_W | Warnschwelle für Alarm A bzw. Warnung W |
| BM, BÖ, KM | Betriebsmittel, Betriebsöl, Kühlmittel |
| MY | Messergebnis, Intensität I |
| MY0 | Messreferenz |
| B | Trendband, |
| $\Delta$B | Fehlerabweichung |
| S | auswertbares Messsignal |
| E, En, E1, E1 | Ereignis, Betriebsölnachfüllung |
| EAn, EA1, EA2 | Anfangsereignis, Betriebsölwechsel |
| R | Referenz-Betriebszeitraum |
| Z | Zustandsmerkmal |
| V | Verlauf |

| Y-1, Y0 | Parametermesswert |
|---|---|
| $\Delta Y$, $\Delta Y0$, S(Delta_I) | einer sprunghaften Änderung zugeordnete Änderungsamplitude bzw. Änderungsamplitude des Messsignals S |
| Delta_I | Intensitätsunterschied |
| lim2 | kleinerer Amplitudenwert |
| lim1 | größerer Amplitudenwert |
| t0 | Zeitpunkt nach dem Ölwechsel, Anfang des Referenzbetriebszeitraums R |
| tR | Ende Referenzbetriebszeitraums R |
| t-1 | Zeitpunkt vor dem Ölwechsel |
| t | Betriebszeit |
| $\Delta t$ | Zeitschritt, Auflösung |
| I_t-1, I_t0, I1t-1, I1t, I2t-1, I2t | Intensitäten |

**Patentansprüche**

1. Verfahren zur Zustandsbestimmung eines in einer Maschine (100) befindlichen Betriebsmittels (BM), insbesondere mit einer Betriebsmittelwechseldetektion oder dergleichen Detektion eines Anfangsereignisses (EAn) oder eines Ereignisses (En), aufweisend die Schritte:

   Zuführen des Betriebsmittels (BM) der Maschine zu einer an die Maschine (100) gekoppelten Spektroskopieeinrichtung (600),
   Durchführen einer spektroskopischen Messanalyse mittels einer Messstrahlung der Spektroskopieeinrichtung (600),
   Bestimmen eines spektroskopischen Messergebnisses (MY) basierend auf der spektroskopischen Messanalyse,
   Setzen des spektroskopischen Messergebnisses (MY) in Relation zu einer Messreferenz (MY0), derart, dass aus der Relation ein auswertbares Messsignal (S) angegeben wird, wobei

   - das spektroskopische Messergebnis (MY) über einen vorbestimmten, auf ein Anfangsereignis (EAn) in Bezug auf das Betriebsmittel (BM) folgenden Referenzbetriebszeitraum (R) wiederholt bestimmt wird unter Angabe von dem Referenzbetriebszeitraum (R) zugeordneten spektroskopischen Messergebnissen (MY), und aus den spektroskopischen Messergebnissen die Messreferenz (MY0) bestimmt wird, und
   - das auswertbare Messsignal (S) über eine Betriebszeit (t) der Maschine (100) wiederholt angegeben wird, derart, dass ein bestimmtes Zustandsmerkmal (Z) für das Betriebsmittel (BM) signalisierbar ist.

2. Verfahren nach Anspruch 1, wobei das Betriebsmittel (BM) in Form eines Betriebsöls (BÖ) und/oder eines Kühlmittels (KM) gebildet ist.

3. Verfahren nach Anspruch 1 oder 2, wobei das spektroskopische Messergebnis (MY) für eine Referenzanzahl von Wiederholungen wiederholt bestimmt wird unter Angabe einer Referenzanzahl von dem Referenzbetriebszeitraum (R) zugeordneten spektroskopischen Messergebnissen (MY) und der auf das Anfangsereignis (EA) in Bezug auf das Betriebsmittel (BM) folgende Referenzbetriebszeitraum (R) so festgelegt wird, dass die Referenzanzahl von spektroskopischen Messergebnissen (MY) im Referenzbetriebszeitraum (R) liegen.

4. Verfahren nach Anspruch 3, wobei geprüft wird, dass für die Referenzanzahl von Wiederholungen das spektroskopische Messergebnis (MY) im Wesentlichen unverändert ist, wobei die Referenzanzahl von spektroskopischen Messergebnissen in einem vorbestimmten Konstantbereich liegt, wobei aus der Referenzanzahl der im Wesentlichen unveränderten spektroskopischer Messergebnisse (MY) des Referenzbetriebszeitraums (R) die Messreferenz (MY0) bestimmt wird.

5. Verfahren nach einem der vorhergehenden Ansprüche, wobei das spektroskopische Messergebnis (MY) zur Verfügung gestellt wird mit Messstrahlung eines vorbestimmten Spektrums für wenigstens eine Messstrahlungslinie, eine Anzahl von Messstrahlungslinien oder für ein Spektrumband der Messstrahlung.

6. Verfahren nach einem der vorhergehenden Ansprüche, wobei das spektroskopische Messergebnis (MY) einem optischen Filter unterworfen wird, und/oder
das auswertbare Messsignal (S) einem numerischen Filter unterworfen wird.

7. Verfahren nach einem der vorhergehenden Ansprüche, wobei das Anfangsereignis (EAn) in Bezug auf das Betriebsmittel (BM) ein zum Bestimmen der Messreferenz (MY0) bestimmtes oder geeignetes Anfangsereignis in Bezug auf das Betriebsmittel ist, das ausgewählt ist aus der Gruppe von: einem Betriebsmittelwechsel, einer Betriebsmittelnachfüllung, insbesondere Betriebsölwechsel (EA1, EA2), Betriebsölnachfüllung (E1, E2).

8. Verfahren nach einem der vorhergehenden Ansprüche, wobei das Anfangsereignis (EAn) in Bezug auf das Betriebsmittel (BM) festgestellt wird, indem geprüft wird, dass das Messsignal (S) für ein Ereigniszeitfenster eine sprunghafte Änderung aufweist.

9. Verfahren nach Anspruch 8, wobei eine der sprunghaften Änderung zugeordnete sprunghafte Änderungsamplitude ($\Delta Y$, S(Delta_I)) einen ersten Ereignis-Schwellwert, insbesondere in Form eines vergleichsweise größeren Amplitudenwerts (lim1), überschreitet, insbesondere das Anfangsereignis oder ein anderes Ereignis als ein Betriebsmittelwechsel, insbesondere Betriebsölwechsel (EA1, EA2), identifiziert wird.

10. Verfahren nach Anspruch 8, wobei eine der sprunghaften Änderung zugeordnete sprunghafte Änderungsamplitude ($\Delta Y$, S(Delta_I)) einen zweiten Ereignis-Schwellwert insbesondere in Form eines vergleichsweise kleineren Amplitudenwerts (lim2), überschreitet, insbesondere das Anfangsereignis oder ein anderes Ereignis als eine Betriebsmittelnachfüllung, identifiziert wird.

11. Verfahren nach Anspruch 9 und 10, wobei der erste Ereignis-Schwellwert den zweiten Ereignis-Schwellwert überschreitet.

12. Verfahren nach einem der vorhergehenden Ansprüche, wobei zum wiederholt angegebenen auswertbaren Messsignal (MY) über die Betriebszeit (t) eine obere und/oder untere Fehlergrenze (oFG, uFG) angegeben wird.

13. Verfahren nach Anspruch 6 und 12, wobei

- das auswertbare Messsignal (S) über die Betriebszeit (t) der Maschine (100) zur Darstellung als Verlauf (V) angegeben wird, wobei mittels des Verlaufs, ein bestimmter Zustand des Betriebsmittels signalisierbar ist, und/oder
- über den Verlauf des auswertbaren Messsignals (S) über die Betriebszeit (t) die obere und/oder untere Fehlergrenze (oFG, uFG) als Fehlergrenzenverlauf angegeben wird, die einen zu bevorzugenden Verlauf des auswertbaren Messsignals (S) vorgeben.

14. Verfahren nach einem der vorhergehenden Ansprüche, wobei das bestimmte Zustandsmerkmal (Z) des Betriebsmittels (BM) signalisierbar ist im Hinblick auf ein Ereignis (En) ausgewählt aus der Gruppe bestehend aus: einem Betriebsmittelwechsel, einer Betriebsmittelnachfüllung, einem Wassereintrag in das Betriebsmittel, einem Kraftstoffeintrag in das Betriebsmittel, einem Eintrag von Verschmutzung und/oder Ruß in das Betriebsmittel.

15. Vorrichtung, vorzugsweise Steuer- und Messvorrichtung (300) für eine Maschine oder Maschine (100) mit der Steuer- und Messvorrichtung, ausgebildet zur Durchführung des Verfahrens nach einem der Ansprüche 1 bis 14.

1000

| 300 | ECU |

REFERENZ  EAn / EREIGNIS, En

400 — S(Delta_I) < lim1          S(Delta_I) < lim2

WARNUNG W / ALARM A

500 — S(Delta_I) < Fehlerband / S(Delta_I) < Alarmschwelle

800
700 — Sens_T          Sens_I — 600

100 — BM ( BÖ, KM)

200

**Fig. 1**

EP 4 685 464 A1

**(A)**

Ölwechsel

$\underline{S}$, V

T,I

MYO — EA — R — MYR

$I_{t0}$

MY

$\Delta I, \Delta Y, \Delta YO$

$I_{t-1}$

$\Delta t$

$Y_{-1}$

$t_{-1}$  $t_0$  $t_R$  t

**(B)**

S1.1 — Start

S1.2 — Messung Intensität $I_t$

S1.3 — $I_t > I_{t-1}$?

S1.4

nein

ja

**Fig.2**

EP 4 685 464 A1

Fig.3

EP 4 685 464 A1

**Fig.4**

S1.1 — Start

S1.2 — Messung Intensität $I_t$

S1.4

S1.3 — $I_t > I_{t-1}$ ?   nein

S4.1

S2.0 — ja

S2.1 — $I_t - I_{t-1} > lim_1$   nein

S3.0

S3.1 — $I_t - I_{t-1} > lim_2$   nein

S2.1 — ja

S3.1 — ja

S2.2 — Ölwechsel, speichere t und $I_t$

Ölnachfüllung, speichere t, $I_t$ und $I_{t-1}$

S4.0

Fig.5

Fig.6B

Fig.6A

Normal zeitliche "Intensitätsalterung" innerhalb ΔB, d.h. der Fehlergrenzen oFG, uFG

Fig.7A

$E_D$, Hinweis auf Motorschaden

Fig.7B

E_R, Hinweis auf Fremdstoffe oder Verrußung

**Fig.8A**

E_L, Hinweis auf Luft

**Fig.8B**

EP 4 685 464 A1

EP 4 685 464 A1

**1000**

**800, 600** — Sens _ I

**BM (BÖ, KM)**

**800, 700** — Sens _ T

**800, 710, 720, 730**

**900**

**Ölsorten-REFERENZ:** Detektion oder Angabe Anfangsereignis, z.B. Ölwechsel-Detektion — EA

**Referenzwert MYO, MYR nach Anfangsereignis, z.B. nach Ölwechsel** — EA

**200**

**300** — ECU

**400** — Ereignis-Detektion, E

**Limit-Überwachung, limA, limW**

**200**

**500**

**A** — ALARM-Funktion: Schwellwert-Signalisierung, z.B. Alarmschwelle SG, SR

**W** — WARN-Funktion: Fehlergrenzen-Signalisierung, z.B. Fehlerband ΔB

**Fig.9**

**Europäisches Patentamt**
**European Patent Office**
**Office européen des brevets**

# EUROPÄISCHER RECHERCHENBERICHT

Nummer der Anmeldung

EP 25 19 1629

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2022/412912 A1 (ZAMBON NATHAN D [US] ET AL) 29. Dezember 2022 (2022-12-29) * Absätze [0003] - [0005], [0137], [0141], [0165], [0167], [0228], [0235] - [0239]; Abbildungen 5,12 * ----- | 1-15 | INV. G01N21/31 G01N21/33 G01N21/3577 G01N21/3581 G01N21/359 |
| A | JP H05 171911 A (CALSONIC CORP; NISSAN MOTOR; KYOSEKI SEIHIN GIJUTSU KENK) 9. Juli 1993 (1993-07-09) * Absätze [0009] - [0010], [0016] * ----- | 1-15 | G01N33/28 |
| A,D | DE 20 2007 019631 U1 (MARTECHNIC GMBH [DE]) 26. August 2014 (2014-08-26) * Absätze [0005], [0013] * ----- | 1-15 | |

RECHERCHIERTE SACHGEBIETE (IPC)

G01N
F01M

Der vorliegende Recherchenbericht wurde für alle Patentansprüche erstellt

| Recherchenort | Abschlußdatum der Recherche | Prüfer |
|---|---|---|
| Den Haag | 3. Dezember 2025 | van Lith, Joris |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 19 1629

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-12-2025

| Im Recherchenbericht angeführtes Patentdokument | Datum der Veröffentlichung | Mitglied(er) der Patentfamilie | Datum der Veröffentlichung |
|---|---|---|---|
| US 2022412912 A1 | 29-12-2022 | CN 117813444 A | 02-04-2024 |
| | | EP 4363700 A1 | 08-05-2024 |
| | | EP 4656849 A2 | 03-12-2025 |
| | | JP 2024525364 A | 12-07-2024 |
| | | KR 20240025622 A | 27-02-2024 |
| | | US 2022412912 A1 | 29-12-2022 |
| | | WO 2023278434 A1 | 05-01-2023 |
| JP H05171911 A | 09-07-1993 | KEINE | |
| DE 202007019631 U1 | 26-08-2014 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

**In der Beschreibung aufgeführte Patentdokumente**

- DE 102020126900 A1

- DE 202007019631 U1